# EUROPEAN PATENT APPLICATION

(11) **EP 3 166 034 A1**
(43) Date of publication of application: **10.05.2017**
(21) Application number: 16190879.3
(22) Date of filing: 27.09.2016
(51) Int. Cl.: G06F 19/00

(54) **MEDICAL APPARATUS, SYSTEM FOR CONTROLLING MEDICAL TEST REQUEST, METHOD FOR CONTROLLING MEDICAL TEST REQUEST, AND PROGRAM STORED IN MEDIUM**

(30) Priority: 03.11.2015 KR 20150153631
(71) Applicant: Samsung Electronics Co., Ltd., Suwon-si, Gyeonggi-do 16677 (KR)
(72) Inventor: Son, Yu Ri, Gyeonggi-do (KR); Shin, Im Ho, Gyeonggi-do (KR); Lee, Chang Sub, Jeollanam-do (KR)
(74) Representative: Hylarides, Paul Jacques

(57) **Abstract**

A medical apparatus, a system for controlling a medical test request, a method for controlling the medical test request, and a program stored in a recording medium are provided. The medical apparatus includes a communication interface configured to receive a test request message requesting a medical test to be performed, and a controller configured to generate a test request reception message based on the received test request message. The communication interface is further configured to transmit the test request reception message to a mobile terminal.

## Description

### BACKGROUND

### 1. Field

Apparatuses and methods consistent with exemplary embodiments relate to a medical apparatus, a system for controlling a medical test request, a method for controlling the medical test request, and a program stored in a recording medium.

### 2. Description of the Related Art

A medical information system (also referred to as a Hospital Information System (HIS)) is an automatic system for automatically performing hospital management or medical management tasks by software. In more detail, the medical information system manages, patient administration information, performs medical prescription management and delivery, performs medical record management and insurance handling, stores/manages/transmits various image data of patients, collects/manages/stores various hospital management information and medical information, and performs various associated tasks. The above-mentioned medical information system can allow doctors, nurses, medical laboratory technologists, and administrators to efficiently and rapidly perform various hospital tasks, and can improve hospital management convenience.

The medical information system connects various computers present in a hospital to server devices through intranet installed in the hospital in a manner that the computers can communicate with the server devices through intranet, such that the medical information system can collect, manage, and store various information related to hospital management or medical tasks.

### SUMMARY

Exemplary embodiments may address at least the above problems and/or disadvantages and other disadvantages not described above. Also, the exemplary embodiments are not required to overcome the disadvantages described above, and may not overcome any of the problems described above.

Exemplary embodiments provide a medical device, a system and a method for controlling a medical test request, and a program stored in a recording medium, which can allow a medical testing staff member located at a remote site to quickly receive/confirm a medical testing request, such that the medical testing can be properly and efficiently performed.

Exemplary embodiments provide a medical device, a system and a method for controlling a medical test request, and a program stored in a recording medium, which can transmit a medical testing request to an appropriate testing staff member, transmit a testing request to a new testing staff member, and quickly and efficiently perform medical testing and observation tasks.

Exemplary embodiments provide a medical device, a system and a method for controlling a medical test request, and a program stored in a recording medium.

According to an aspect of an exemplary embodiment, there is provided a medical apparatus including a communication interface configured to receive a test request message requesting a medical test to be performed, and a controller configured to generate a test request reception message based on the received test request message. The communication interface is further configured to transmit the test request reception message to a mobile terminal.

The test request reception message may include at least one among information regarding an object to be tested, information regarding a specimen, information regarding the requested medical test, information regarding a pre-stored medical test, information regarding an order of the requested medical test and the pre-stored medical test, information regarding a status of the medical apparatus, information regarding an urgent test request, information regarding a test requesting person, and information regarding an examiner who conducts a test.

The controller may be further configured to determine an order of medical test requests based on the test request message, and change the order based on information regarding an urgent medical test request or a command for changing the order that is received from the mobile terminal.

The controller may be further configured to determine an estimated standby time based on the received test request message.

The controller may be further configured to determine the estimated standby time based on a number of estimated medical tests and a category of an estimated medical test, or based on the number of the estimated medical tests, the category of the estimated medical test, and a status of the medical apparatus.

The controller may be further configured to correct an order of medical test requests based on an urgency degree of each of the medical test requests, and determine the estimated standby time based on the corrected order.

The controller may be further configured to determine the mobile terminal to which the test request reception message is transmitted, and the communication interface may be further configured to transmit the test request reception message to the determined mobile terminal.

The controller may be further configured to determine the mobile terminal to which the test request reception message is transmitted, based on a priority of each of examiners, and the communication interface may be further configured to transmit the test request reception message to a high-priority mobile terminal of an examiner having a highest priority, among mobile terminals of the examiners.

The controller may be further configured to determine the priority based on at least one among a medical test history of each of the examiners, a number of medical tests that are conducted by each of the examiners, a number of medical tests to be conducted by each of the examiners, priority information, and a user input.

The communication interface may be further configured to receive, from the mobile terminal, a request acceptance message indicating acceptance of the test request reception message or a request denial message indicating denial of the test request reception message.

The controller may be further configured to, based on the received request denial message, or in response to the communication interface not receiving a response signal from the mobile terminal for a time, control the communication interface to transmit the test request reception message to a subsequent-priority mobile terminal of an examiner having a priority subsequent to the highest priority, among the mobile terminals.

The controller may be further configured to, based on the received request acceptance message, and in response to medical testing not starting for a time, control the communication interface to transmit the test request reception message to a subsequent-priority mobile terminal of an examiner having a priority subsequent to the highest priority, among the mobile terminals.

The request denial message may include an additional message of a user of the mobile terminal.

The communication interface may be further configured to receive, from the mobile terminal, a transmission command to transmit the test request reception message from the mobile terminal to another mobile terminal, and the controller may be further configured to control the communication interface to transmit the test request reception message to the other mobile terminal based on the received command.

The mobile terminal may include the test request reception message, and transmits the test request message corresponding to the test request reception message to another medical apparatus.

The communication interface may be further configured to receive the test request message from one among a personal computer, a server, and a mobile terminal.

The controller may be further configured to determine information as to whether it is impossible to use the medical apparatus, and the communication interface may be further configured to transmit the information to the mobile terminal.

The controller may be further configured to perform an authentication prior to the transmission of the test request reception message.

The controller may be further configured to perform the authentication based on identification information of the mobile terminal or identification information of a user of the mobile terminal.

According to an aspect of an exemplary embodiment, there is provided a method for controlling a medical test request, the method including receiving, by a medical apparatus, a test request message requesting a medical test to be performed, generating, by the medical apparatus, a test request reception message in response to the receiving the test request message, and transmitting, by the medical apparatus, the test request reception message to a mobile terminal.

According to an aspect of an exemplary embodiment, there is provided a medical apparatus including a communication interface configured to receive a request for a medical test, and a controller configured to determine an order of requests for medical tests including the received request, and determine an examiner to perform a next medical test, based on the determined order. The communication interface is further configured to transmit, to a mobile terminal of the determined examiner, a request for the determined examiner to perform the next medical test.

The received request may include a date and a time of the received request, an identification of a patient to which the medical test is to be performed, information of equipment to be used in the medical test, and an examiner to perform the medical test.

The controller may be further configured to determine the order based on priorities of examiners corresponding to the respective requests, and determine, as the examiner, an examiner corresponding to a request highest in the determined order.

### BRIEF DESCRIPTION OF THE DRAWINGS

The above and/or other aspects will be more apparent by describing exemplary embodiments with reference to the accompanying drawings, in which:
FIG. 1 is a conceptual diagram illustrating an overall structure of a medical testing request control system according to an exemplary embodiment;
FIG. 2 is a perspective view illustrating the external appearance of an in vitro diagnosis (IVD) medical device among various medical devices, according to an exemplary embodiment;
FIG. 3 is a block diagram illustrating a medical device according to an exemplary embodiment;
FIG. 4 is a block diagram illustrating a mobile terminal device according to an exemplary embodiment;
FIG. 5 is a diagram illustrating a list of observation (or testing) requests according to an exemplary embodiment.
FIG. 6 is a conceptual diagram illustrating a method for deciding a terminal device according to whether or not medical testing is pre-performed, according to an exemplary embodiment.
FIG. 7 is a conceptual diagram illustrating a method for deciding a terminal device according to a number of medical tests or a number of residual tests, according to an exemplary embodiment.
FIG. 8 is a conceptual diagram illustrating a list of medical testing staff member priorities according to a decision result, according to an exemplary embodiment.
FIG. 9 is a conceptual diagram illustrating a first screen image through which medical testing staff member priority is manually changed, according to an exemplary embodiment.
FIG. 10 is a conceptual diagram illustrating a second screen image through which medical testing staff member priority is manually changed, according to an exemplary embodiment.
FIG. 11 is a conceptual diagram illustrating a method for transmitting a test request reception message to a mobile terminal device, according to an exemplary embodiment.
FIG. 12 is a conceptual diagram illustrating a method for displaying a test request reception message to a mobile terminal device, according to an exemplary embodiment.
FIG. 13 is a conceptual diagram illustrating a method for allowing a medical testing staff member to accept a test request, according to an exemplary embodiment.
FIG. 14 is a conceptual diagram illustrating a list of medical test requests when a medical testing staff member accepts a test request, according to an exemplary embodiment.
FIG. 15 is a conceptual diagram illustrating a method for allowing a medical testing staff member to reject a test request, according to an exemplary embodiment.
FIG. 16 is a conceptual diagram illustrating a method for transmitting a test request reception message to a second mobile terminal device according to rejection of the test request, according to an exemplary embodiment.
FIG. 17 is a conceptual diagram illustrating a method for checking a list of medical tests using a mobile terminal device, according to an exemplary embodiment.
FIG. 18 is a conceptual diagram illustrating a first method for deciding an order of tests upon receiving an urgent test request, according to an exemplary embodiment.
FIG. 19 is a conceptual diagram illustrating a second method for deciding an order of tests, according to an exemplary embodiment.
FIG. 20 is a conceptual diagram illustrating a method for changing an order of tests according to an exemplary embodiment;
FIG. 21 is a conceptual diagram illustrating a method for changing an order of tests according to an exemplary embodiment;
FIG. 22 is a conceptual diagram illustrating a first method for changing an order of tests using a mobile terminal device, according to an exemplary embodiment.
FIG. 23 is a conceptual diagram illustrating a second method for changing an order of tests using a mobile terminal device, according to an exemplary embodiment.
FIG. 24 is a conceptual diagram illustrating a third method for changing an order of tests using a mobile terminal device, according to an exemplary embodiment.
FIG. 25 is a conceptual diagram illustrating a first method for calculating an estimated standby time, according to an exemplary embodiment.
FIG. 26 is a conceptual diagram illustrating a second method for calculating an estimated standby time, according to an exemplary embodiment.
FIG. 27 is a conceptual diagram illustrating a third method for calculating an estimated standby time, according to an exemplary embodiment.
FIG. 28A is a conceptual diagram illustrating a method for checking states of a plurality of medical devices using a mobile terminal device, according to an exemplary embodiment.
FIG. 28B is a conceptual diagram illustrating a method for correcting medical test request lists respectively stored in respective medical devices, according to an exemplary embodiment.
FIG. 28C is a diagram illustrating a screen image for displaying a number of medical test request lists respectively stored in respective medical devices displayed on a mobile terminal device, according to an exemplary embodiment.
FIG. 28D is a diagram illustrating a screen image for displaying a number of medical test request lists respectively stored in respective medical devices displayed on a mobile terminal device, according to an exemplary embodiment.
FIG. 28E is a diagram illustrating a screen image for correcting medical test request lists, according to an exemplary embodiment.
FIG. 29 is a flowchart illustrating a method for authenticating a mobile terminal device, according to an exemplary embodiment.
FIG. 30 is a diagram illustrating an authentication screen image displayed on a mobile terminal device, according to an exemplary embodiment.
FIG. 31 is a conceptual diagram illustrating a first method for selecting a medical test to be requested for another medical testing staff member using a mobile terminal device, according to an exemplary embodiment.
FIG. 32 is a conceptual diagram illustrating a second method for selecting a medical test to be requested for another medical testing staff member using a mobile terminal device, according to an exemplary embodiment.
FIG. 33 is a conceptual diagram illustrating a third method for selecting a medical test to be requested for another medical testing staff member using a mobile terminal device, according to an exemplary embodiment.
FIG. 34 is a flowchart illustrating a first method for controlling a medical test request, according to an exemplary embodiment;
FIG. 35 is a flowchart illustrating a second method for controlling a medical test request, according to an exemplary embodiment;
FIG. 36 is a flowchart illustrating a method for calculating an estimated standby time, according to an exemplary embodiment;
FIG. 37 is a flowchart illustrating a method for transmitting a test request reception message to another test staff member, according to an exemplary embodiment.

### DETAILED DESCRIPTION

Exemplary embodiments are described in greater detail below with reference to the accompanying drawings.

In the following description, like drawing reference numerals are used for like elements, even in different drawings. The matters defined in the description, such as detailed construction and elements, are provided to assist in a comprehensive understanding of the exemplary embodiments. However, it is apparent that the exemplary embodiments can be practiced without those specifically defined matters. Also, well-known functions or constructions are not described in detail because they would obscure the description with unnecessary detail.

It will be understood that the terms such as "unit," "-er (-or)," and "module" described in the specification refer to an element for performing at least one function or operation, and may be implemented in hardware, software, or the combination of hardware and software.

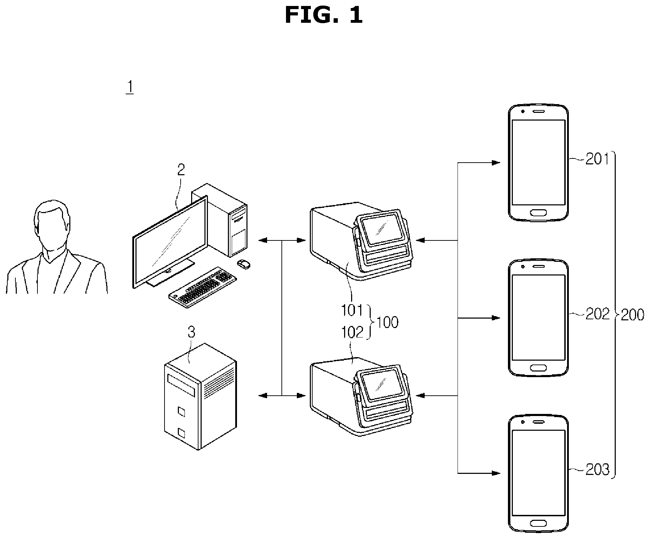

FIG. 1 is a conceptual diagram illustrating an overall structure of a medical testing request control system according to an exemplary embodiment.

Referring to FIG. 1, a medical test request control system 1 may include at least one computing device 2, at least one medical device 100, and at least one terminal device 200. For convenience of description, although the medical test request control system 1 according to an exemplary embodiment may include two medical devices (101, 102) and three terminal devices (201, 202, 203) for convenience of description, the number of medical devices 100 and the number of the terminal devices 200 are not limited thereto. In accordance with an exemplary embodiment, only one medical device 100 may be used, or three or more medical devices 100 may be used. In addition, one or more terminal devices 200 may be used, or four or more terminal devices 200 may be used. In addition, one among plural medical devices (101, 102) is referred to as a first medical device 101, and the other one is referred to as a second medical device 102. A first one among plural medical devices (201, 202, 203) is referred to as a first terminal device 201, a second one is referred to as a second terminal device 202, and a third one is referred to as a third terminal device 203. However, numerals added to the medical devices and the terminal devices do not indicate special orders, are simply added to identify respective entities, and may be randomly added according to system designers.

The computing device 2, at least one medical device 100, and at least one terminal device 200 may communicate with one another through a wired or wireless communication network.

The wired communication network may be constructed through a cable connected to each device. The cable may include a pair cable, a coaxial cable, an optical fiber cable, or an Ethernet cable.

The wired communication network may be implemented using a local area network (LAN) or a mobile communication network. The local area network (LAN) may be implemented using Wireless LAN, Wi-Fi, Bluetooth, ZigBee, CAN communication, Wi-Fi Direct (WFD), Ultra wideband (UWB), Infrared Data Association (IrDA), Bluetooth Low Energy (BLE), Near Field Communication (NFC), etc. The mobile communication network may also be implemented using any of mobile communication protocols, for example, 3GPP, 3GPP2, World Interoperability for Microwave Access (WiMAX), etc.

The computing device 2 and at least one medical device 100 may communicate with each other using various communication protocols, such that the computing device 2 can transmit/receive various medical information, test request messages, etc. to/from the at least one medical device 100. In this case, protocols (HL7, ASTM, or POCT1A) may be used.

The computing device 2 may receive test request messages from a desktop computer, laptop, netbook, tablet PC, smartphone, mobile phone, etc. The computing device 2 may be implemented using various devices capable of transmitting the received test request messages to the medical device 100 or the server device 3. In accordance with an exemplary embodiment, the number of computing devices 2 for use in the medical test request control system 1 may not be specially limited thereto. For example, one or more computing devices 2 may be used.

The computing device 2 may receive various commands or information related to a test request for a specimen from the test requesting staff member. In this case, the test requesting staff member may include doctors, nurses, medical device operators, who can request a medical test for the patient to be diagnosed. The specimen may be a material or living thing to be tested or analyzed, for example, an environmental sample, a biological sample, a food sample, etc. For example, the target object may include all or some parts of a human body, cellular tissues or blood separately extracted from the target object, and various materials such as saliva or urine. In this case, the target object to be tested may be a specimen, or may be a human being or animal from which the specimen is extracted. The specimen may be any one among a fluid, a solid, etc.

The test requesting person (staff member) may manipulate various input devices, for example, a keyboard, a mouse, a touchscreen, a touchpad, a trackball, etc., mounted to the computing device, and may input a test request for the specimen and other information related to the test request.

After the computing device 2 generates a test request message on the basis of the input test request or various information, the computing device 2 may transmit the generated test request message to one or more medical devices 100 through a wired or wireless communication network. To communicate with the medical device 100, the computing device may include a communication interface therein. The communication interface may include at least one among a wired communication interface to access the above-mentioned wired communication network and a wireless communication interface to access the wireless communication network.

The test request message may include various kinds of information associated with tests. The test-associated information may include at least one among information regarding a target person to be tested, information regarding a specimen, information regarding the requested test, information regarding the presence or absence of an urgent test, information regarding the test requesting person, and information regarding the testing staff member. In this case, the testing staff member may be a person (e.g., a medical laboratory technologist) who tests and analyzes the specimen using the medical device 100.

The computing device 2 may transmit a test request message to a medical device selected by the testing staff member, among the plurality of medical devices (101, 102). In this case, assuming that the testing staff member selects only one medical device (e.g., a first medical device 101), if the computing device 2 may transmit a test request message only to the selected first medical device 101, and if the test requesting person selects the plurality of medical devices (101, 102), the test request message can be transmitted to all the selected medical devices, i.e., the first medical device 101 and the second medical device 102.

The computing device 2 may also transmit a test request message to one medical device 100 or several medical devices (101, 102) according to the predefined setting stored in the computing device 2.

According to an exemplary embodiment, the computing device 2 may directly transmit a message corresponding to a test request command to one or more medical devices 100. According to another exemplary embodiment, the computing device 2 may transmit the message to one or more medical devices 101 through a separate server device 3.

The separate server device 3 may transmit the message received from the computing device 2 to the medical device 100. Upon receiving information regarding the medical device 100 from the computing device 2, the server device 3 may select at least one medical device 100 from the plurality of medical devices 100, and may transmit the message to the selected medical device 100.

The server device 3 may be implemented using various devices each functioning as a server, and may also be implemented using a server computer, a desktop computer, or the like.

The server device 3 may be omitted for convenience of description. In this case, the test request message may be directly transferred from the computing device 2 of the testing requesting person to the medical device 100. If the server device 3 is omitted as described above, a device to be used as the separate service device 3 need not be purchased or a space in which the device will be disposed need not be separately provided, such that the medical test request control system 1 can be simply and quickly installed at less cost.

The medical device 100 may be a device configured to perform various inspections for a specimen as well as to analyze and test the specimen. The medical device 100 may include various electronic components to perform various inspections on the specimen, and may also include communication associated components to implement data communication between the computing device 2 and the terminal device 200.

The medical device 100 may receive the test request message from the computing device 2 using the communication associated components, may generate a test request reception message on the basis of the received test request message, and may transmit the test request reception message to the terminal device 200.

For example, the medical device 100 may include in vitro diagnosis (IVD) medical devices and image diagnosis medical devices. Besides, the medical device 100 may include various devices or machines used when the specimen is tested in hospitals or schools.

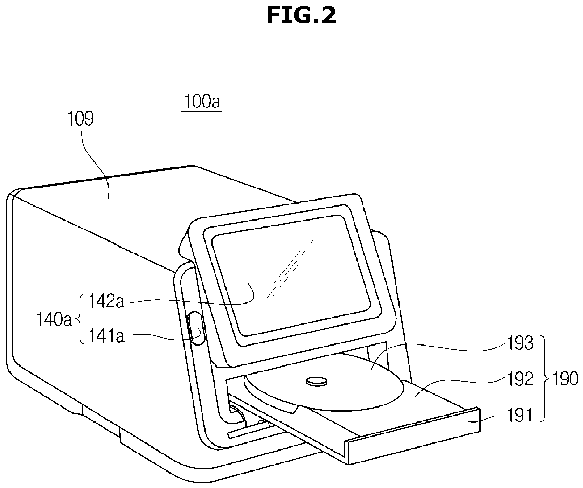

FIG. 2 is a perspective view illustrating the external appearance of an IVD medical device among various medical devices, according to an exemplary embodiment.

An IVD medical device 100a may test and analyze tissues, blood, or secretions obtained by the testing staff member or from the patient to be tested, thereby obtaining the medical test result. The IVD medical device may include various devices to test the specimen in various technical fields, for example, immunochemistry diagnostics, blood sugar measurement, Point of Care Testing (POCT), molecular diagnostics, hematology, clinical microbiology, hemostasis, tissue diagnostics, etc. For example, the IVD medical device may include various diagnostic kits, microfluidic devices, or the like.

Referring to FIG. 2, the IVD medical device 100a includes an external housing 109, a user interface 140a, and an analysis portion 190.

The external housing 109 may achieve the external appearance of the IVD medical device 100a, and the user interface 140a may be located at the outside the external housing 109. In addition, the external housing 109 may be designed to expose some components (e.g., a tray 192) of the external analysis portion 190. A component to analyze the specimen, a component to communicate with the external computing device 2 or the terminal device 200, and other components (i.e., a semiconductor chip, substrate, or antennas) to perform various calculation processes used to operate the IVD medical device 100a may be installed in the external housing 109. The components such as semiconductor chips may serve as a communication interface 110, a controller 120, a storage 130, etc. to be described later.

The user interface 140a may receive various commands or information from a user (e.g., a testing staff member), and may provide the received information to the user. The user interface 140a includes an input interface 141a to receive a user command or information, and a display 142a to provide the received information to the user.

The IVD medical device 100a may include an analysis portion 190 to analyze a specimen. The analysis portion 190 includes a reaction portion 193 and a sensor to detect a reaction generated from the reaction portion 193.

The reaction portion 193 may include a fluid specimen such as blood, and a biochemical reaction of the fluid specimen occurs in the reaction portion 193. For example, the reaction portion 193 may be formed in a disc shape as shown in FIG. 2. The disc-shaped reaction portion 193 may include a platform forming the disc, and a plurality of chambers formed on the platform. The specimen and various reagents may be injected into the plurality of chambers. In accordance with an exemplary embodiment, the reaction portion may also be formed in a cartridge shape. The disc-shaped reaction portion 193 or the cartridge-shaped reaction portion may be detachably connected to all the IVD medical devices 100a.

If the reaction portion 193 is formed in a disc shape, the analysis portion 190 may include a tray 191, the tray 192 on which the reaction portion 193 is seated, and a driver to rotate the reaction portion 193, such that the reaction portion 193 is inserted into the analysis portion 190 and then rotates therein.

The tray 192 may be configured to be exposed to the outside according to manipulation by user, e.g., the testing staff member, and may be again inserted into the external housing 109 according to user manipulation. A tray cover 191 may protect the tray 192 from external impact or foreign material, and prevent the reaction portion 193 mounted to the tray 192 from being arbitrarily discharged to the outside. The tray cover 191 may be omitted. If the driver is embedded in the external housing 109 and the tray 192 is completely inserted into the external housing 109, the reaction portion 193 seated on the tray 192 rotates.

The reaction portion 193 is seated on the tray 192, is inserted into the external housing 109, and rotates according to driving of the driver. Therefore, the fluid specimen accommodated in the reaction portion 193 is subjected to various reactions. As described above, the biochemical reaction generated in the reaction portion 193 may be detected by a sensing device embedded in the external housing 109. The sensing device may detect a biochemical reaction using optical signals or light.

The image diagnosis medical device may capture the external or internal part of a target person (i.e., patient) using radiation, ultrasonic waves, or magnetic resonance imaging (MRI), such that it obtains images regarding the external or internal part of the target person. The image diagnosis medical device may include a digital radiation imaging apparatus, a mammography apparatus, a computed tomography (CT) apparatus, a magnetic resonance imaging (MRI) apparatus, etc.

The medical device according to an exemplary embodiment will hereinafter be described with reference to the attached drawings.

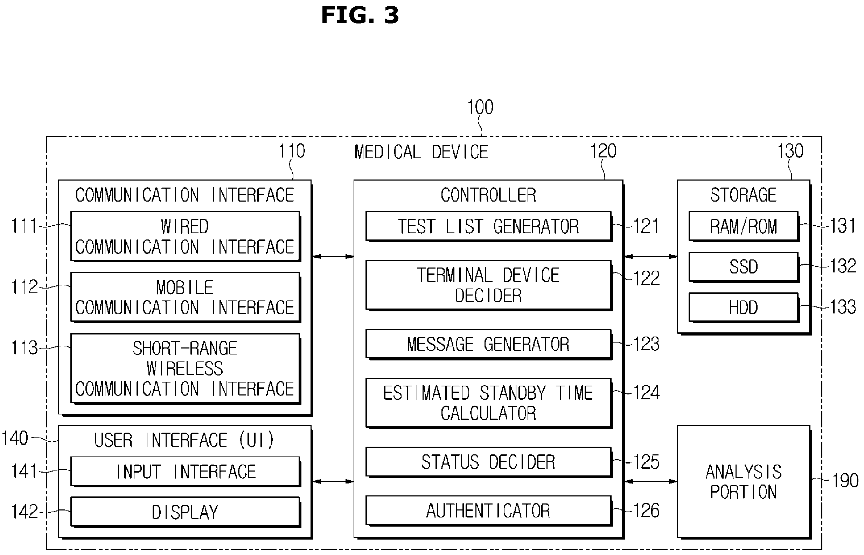

FIG. 3 is a block diagram illustrating a medical device according to an exemplary embodiment. Referring to FIG. 3, a medical device 100 includes a communication interface 110, a controller 120, a storage 130, a user interface 140, and an analysis portion 190. The communication interface 110, the controller 120, the storage 130, the user interface 140, and the analysis portion 190 may communicate with one another through cables, circuits or radio frequency (RF) communication interfaces respectively embedded therein. Accordingly, the communication interface 110, the controller 120, the storage 130, the user interface 140, and the analysis portion 190 may communicate with one another such that various data or commands are communicated therebetween. Besides, the medical device 100 may further include other components used for operation thereof.

The communication interface 110 may communicate with other devices, for example, communication interfaces respectively embedded in the computing device 2 and the terminal device 200. Therefore, the medical device 100 may receive various kinds of information or commands from the computing device 2 and the terminal device 200, or may transmit various information or commands to the devices (2, 200).

The communication interface 110 may include a wired communication interface 111 to access the wired communication network. The wired communication interface 111 may include a communication port capable of being connected to a separate cable, and various units to modulate, amplify, or transmit electrical signals received through the communication port. In this case, the various units may be implemented using a semiconductor chip, a substrate, a circuit, etc.

The communication interface 110 may include at least one among a mobile communication interface 112 and a short-range wireless communication interface 113. The mobile communication interface 112 may perform communication using various mobile communication standards, for example, 3GPP, 3GPP2, WiMAX, etc. The mobile communication interface 112 may wirelessly communicate with the terminal devices (200, 201) located at a remote site or the computing device 2. The short-range wireless communication interface 113 may perform communication using a short distance communication standard such as Wi-Fi or Bluetooth, and may wirelessly communicate with the terminal devices (200, 201) or the computing device 2 located at a short distance therefrom. The mobile communication interface 112 and the short-range wireless communication interface 113 may be implemented using an antenna to transmit or receive radio frequency (RF) signals, a semiconductor chip to perform various processes on electric signals received through the antenna, a substrate on which the semiconductor chip is installed, and various components used for wireless communication.

At least one among the wireless communication interface 111, the mobile communication interface 112, and the short-range wireless communication interface 113 may be omitted.

The communication interface 110 may directly receive a test request message from the computing device 2 as described above, and may receive the test request message transmitted from the computing device 2 through the server device 3 according to an exemplary embodiment. The test request message may be transmitted to the controller 120, may be temporarily or non-temporarily stored in the storage 130, and may then be transmitted to the controller 120.

The controller 120 is configured to control overall operations of the medical device 100. In more detail, the controller 120 may control the communication interface 110, the storage 130, the user interface 140, and/or the analysis portion 190, or may generate a new command or information by fabricating various commands or information acquired from the above components (110, 130, 140, 190). Then, the controller 120 may transmit the new command or information to the respective components (110, 130, 140, 190).

The controller 120 may be implemented using at least one semiconductor chip mounted to the substrate and various auxiliary components of the semiconductor chip.

According to an exemplary embodiment, the controller 120 includes a test list generator 121, a terminal device decider 122, a message generator 123, an estimated standby time calculator 124, a status decider 125, and an authenticator 126. In accordance an exemplary embodiment, some parts of the above-mentioned units (121 to 126) may be omitted.

The test list generator 121, the terminal device decider 122, the message generator 123, the estimated standby time calculator 124, the status decider 125, and the authenticator 126 may be physically or logically separated from one another. If the above-mentioned units (121 to 126) are physically separated from one another, the test list generator 121, the terminal device decider 122, the message generator 123, the estimated standby time calculator 124, the status decider 125, and/or the authenticator 126 may be implemented using the plurality of physically separated semiconductor chips and associated components. If the above-mentioned units (121 to 126) are logically separated from one another, the test list generator 121, the terminal device decider 122, the message generator 123, the estimated standby time calculator 124, the status decider 125, and/or the authenticator 126 may be implemented using one or more semiconductor chips and associated components.

The test list generator 121, the terminal device decider 122, the message generator 123, the estimated standby time calculator 124, the status decider 125, and the authenticator 126 will hereinafter be described in detail.

The storage 130 may temporarily or non-temporarily store various kinds of information used to operate the medical device 100. For example, the storage 130 may store information regarding the plurality of terminal devices (201 to 203). In this case, each of the terminal devices (201 to 203) may be connected to the medical device 100.

In more detail, the storage 130 may store identifiable numbers (e.g., Internet Protocol (IP) addresses or phone numbers) respectively assigned to the terminal devices 201, 202, and 203. The storage 130 may further store information regarding a user (e.g., a testing staff member who handles the medical device 100) corresponding to each terminal device 201, 202, or 203. In this case, the testing staff member corresponding to each terminal device 201, 202 or 203 may be a testing user who uses each terminal device 201, 202 or 203.

In another example, the storage 130 may store a test request list 10 generated in the test list generator 121, the list of terminated tests, and various statistical documents regarding the terminated tests. In addition, the storage 130 may store operations of the medical device 100 and various kinds of information used for medical test request control.

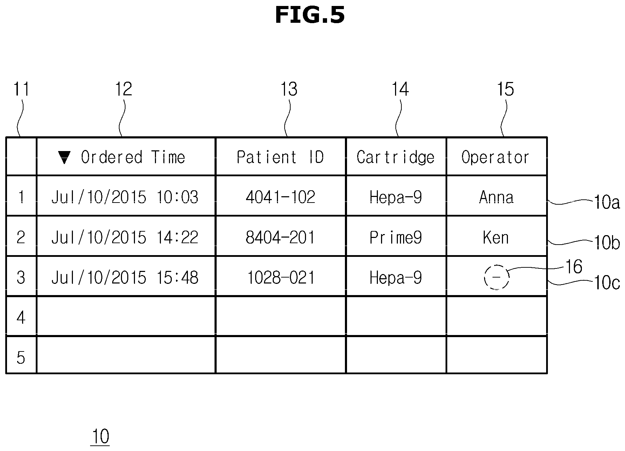

The storage 130 may include a main memory 131 to assist the operation of the controller 120, and auxiliary memories (132, 133) to store various kinds of information such as authentication information or the test request list 10 (see FIG. 5). The storage 130 may include a Random Access Memory (RAM) or Read Only Memory (ROM) acting as a main memory device. For example, the RAM may include a Dynamic Random Access Memory (RAM), a Static Random Access Memory (SRAM), etc. For example, the ROM may include an Erasable Programmable Read-Only Memory (EPROM), an Electrically Erasable Programmable Read-Only Memory (EEPROM), a Mask ROM (MROM), etc. Representative examples of the auxiliary memories contained in the storage 130 may include a Solid State Drive (SSD) 132 to store information using a semiconductor, and a Hard Disc Drive (HDD) 133 to store information using a magnetic disc. Additionally, the auxiliary memories contained in the storage 130 may further include various kinds of storage media, for example, a compact disc (CD), a laser disc, a magnetic tape, a magneto-optical disc, a floppy disc, etc.

The user interface 140 may receive various commands or information from a user, or may provide various kinds of information to the user. The user interface 140 may include an input interface 141 and a display 142, may receive various commands or information from the user through the input interface 141 and the display 142, or may provide various kinds of information to the user.

The input interface 141 may be implemented using a physical button, a keyboard, a touchpad, a touchscreen, a knob, a manipulation stick, a trackball, a track pad, a mouse, or the like. The display 142 may be implemented using various display panels such as a liquid crystal display panel or an organic light emitting diode (OLED) display panel, or using various lighting units such as a light emitting diode (LED). In addition, the display 142 may also be implemented using a touchscreen. If the display 142 is implemented using the touchscreen, the display 142 may function as the above-mentioned input interface 141. In this case, the input interface 141 may also be omitted.

In addition, the user interface 140 may include a sound output interface configured to output a voice or sound signal, for example, a speaker, a headset, or an earphone, or the like. Additionally, the user interface 140 may further include various devices configured to perform interaction between the user and the medical device 100.

The analysis portion 190 may be configured to test or analyze the specimen. For example, assuming that the medical device 100 is identical to the IVD medical device 100a, the analysis portion 190 may include a tray 19a, a reaction portion 193, a drive unit, a sensor, etc., as shown in FIG. 2. In addition, the analysis portion 190 may further include various electronic components according to categories of the medical device 100.

If the medical device 100 is identical to the image diagnosis medical device, the medical device 100 may include an image capturing device instead of the above-mentioned analysis portion 190. If the medical device 100 is identical to the radiographic imaging device, the image capturing device may include a radiation source, a detector, etc. If the medical device 100 is identical to the ultrasonic diagnosis device, the image capturing device may include an ultrasonic probe, various devices for beamforming, etc. If the medical device 100 is identical to the MRI device, the image capturing device may include a static field coil, a gradient field coil, a radio frequency (RF) coil, an image processing unit, etc.

The terminal device 200 will hereinafter be described in detail.

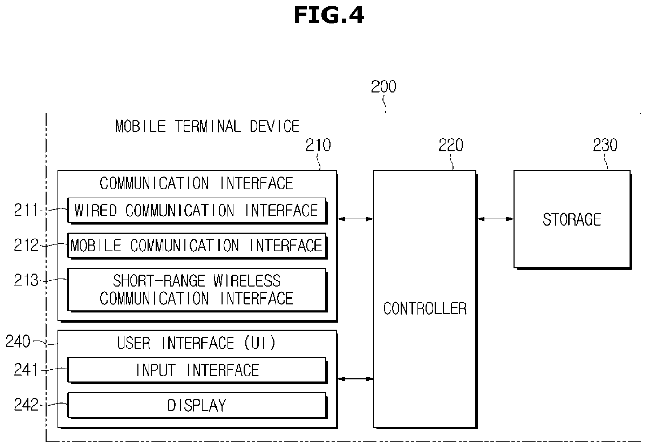

FIG. 4 is a block diagram illustrating a mobile terminal device according to an exemplary embodiment.

Referring to FIG. 4, a mobile terminal device 200 may be configured to communicate with the medical device 100, may receive various commands or information from the medical device 100, or may transmit various commands or information entered by a user (e.g., a testing staff member) of the terminal device 200 to the medical device 100.

The terminal device 200 may be a mobile terminal device, for example, a laptop, a smartphone, a cellular phone, a tablet PC, a navigation device, a portable gaming system, a Personal Digital Assistant (PDA), an electronic notebook, or the like.

As can be seen from FIG. 4, the portable terminal device 200 includes a communication interface 210, a controller 220, a storage 230, and a user interface 240.

The communication interface 210 communicates with the medical device 100 such that the portable terminal device 200 transmits or receives various commands and information to or from the medical device 100. The communication interface 210 may include a wired communication interface 111 to access the above-mentioned wired communication network, and may include a mobile communication interface 212 to perform communication using various mobile communication standards, for example, 3GPP, 3GPP2, WiMAX, etc. In addition, the communication interface 210 may include a short distance communication interface 213 to perform communication using the short distance communication standards, for example, Wi-Fi, Bluetooth, etc. At least one among the wired communication interface 211, the mobile communication interface 212, and the short-range wireless communication interface 213 may be omitted according to categories or characteristics of the terminal device 200. For example, the smartphone or the tablet PC may not include the wired communication interface 211.

The controller 220 may control overall operations of the terminal device 200. In more detail, the controller 220 may display information transmitted through the communication interface 210 for user recognition by controlling a display 242. In addition, the controller 220 may process commands or information entered through an input interface 241, and may provide the processed commands or information to the medical device 100. The controller 220 may be implemented using one or more semiconductor chips and associated components.

The storage 230 may temporarily or non-temporarily store various kinds of information used to operate the terminal device 200. The storage 230 may include a main memory and an auxiliary memory. The storage 230 may be implemented using a semiconductor storage, a magnetic disc storage, or the like.

The user interface 240 may receive various commands or information from the user of the terminal device 200 or provide various kinds of information to the user. For this purpose, the user interface 240 includes the input interface 241 and the display 242.

The input interface 241 may be implemented using a physical button, a keyboard, a touchpad, a touchscreen, a knob, a manipulation stick, a trackball, a track pad, a keypad, a mouse, or the like. The display 242 may be implemented using a display panel or various lighting units, or using a touchscreen. If the display 242 is implemented using the touchscreen, the display 242 may function as the input interface 241. In this case, the input interface 242 may be omitted according to selection of a system designer.

The user interface 240 may further include various devices, for example, a sound output interface configured to output a voice or sound signal.

The portable terminal device 200 may further include an Input/Output (I/O) port, for example, a Universal Serial Bus (USB) port, a micro-USB port, or the like. In this case, the portable terminal device 200 may further receive various information using the above port or may output various information to the external part using the above port.

The controller 120 of the medical device 100 will hereinafter be described with reference to FIGS. 5 to 33.

As drawn in FIG. 3, the controller 120 may include a test list generator 121, a terminal device decider 122, a message generator 123, an estimated standby time calculator 124, a status decider 125, and an authenticator 126.

FIG. 5 is a diagram illustrating a list of observation (or testing) requests according to an exemplary embodiment.

The test list generator 121 may collect the transmitted test request messages through the communication interface 110, and may generate a test request list 10 shown in FIG. 5.

The generated test request list 10 may include one or more pieces of test request information 10a to 10c configured to construct the test request list 10 as shown in FIG. 5. Respective test request information 10a to 10c may correspond to respective test requests. In more detail, the test request information 10a to 10c may be generated on the basis of the transmitted test request message.

For example, the respective test request information 10a to 10c may include various items, for example, a test order 11, a test request time 12, an identification code 13 of a target person to be tested, category information 14 of a disc-shaped or cartridge-shaped reaction portion, and an identification code 15 of the testing staff member who performs the test. In addition, each test request information 10a, 10b, or 10c may further include various items, for example, information regarding the target person to be tested, information regarding the specimen, information regarding the requested test category, test requesting person information including an identification code of the test requesting person, etc. Each item contained in each test request information 10a, 10b, or 10c may be arbitrarily selected by a system designer, and may be selected by a user (e.g., the testing staff member) of the medical device 100. Each item contained in each test request information 10a, 10b, or 10c may be added, deleted, or changed according to manipulation by the testing staff member.

The test list generator 121 may generate the test request list 100 including a single piece of test request information using a new test request message, when a legacy test request list 10 is not present. When the legacy test request list is present, the test list generator 121 inserts test request information corresponding to the new test request message into the legacy test request list 10, updates the test request list 10, and thus generates the test request list 10 including a plurality of pieces of test request information.

The test list generator 121 may generate the test request list 10 by adding the test order 11 to the test request information such that testing is performed according to the reception order of the test request messages. In more detail, the test list generator 121 may determine first test request information 10a corresponding to a first test request message to be test request information indicating a first test order 11, and may determine second test request information 10b corresponding to the second test request message to be test request information indicating a second test order 11. In order words, if the stored test request list 10 is present, the test order 11 is assigned to test request information corresponding to the new test request message in a manner that the test request information can be finally tested. In this case, as shown in FIG. 5, each test request information 10a to 10c of the test request list 10 may be arranged according to the test order 11 in a manner that the user such as the testing staff member can easily recognize the test order.

If the new test request message is transmitted, the testing staff member is not decided, such that the testing staff member identification code 15 of the new test request information 10c may be an empty space 16. The empty space 16 of the testing staff member identification code 15 may be decided by the terminal device decider 122.

The terminal device decider 122 may determine one or more testing staff members, who will transmit the test request, among the plurality of testing staff members. Because each testing staff member uses his or her terminal device 200, the terminal device decider 122 determines one or more terminal devices 200, who will transmit the test request reception message generated by the message generator 123, among the plurality of terminal devices 200. In other words, the terminal device decider 122 may determine a terminal device (e.g., a first terminal device 201) of the testing staff member who is scheduled to perform the test among the plurality of terminal devices (201 to 203).

According to an exemplary embodiment, the terminal device decider 122 may read the test request message to determine the terminal device 201 of the scheduled testing staff member, such that it can determine one or more terminal devices 200 scheduled to transmit the test request reception message on the basis of any one among information regarding the testing requesting person, information regarding the testing staff member designated by the server device 3, and/or information regarding the terminal devices (201 to 203) corresponding to the testing staff member. In this case, the terminal device decider 122 may determine one or more terminal devices 200 scheduled to transmit the test request reception message only to one terminal device 200 corresponding to the test requesting person or the testing staff member designated by the server device 3. In this case, the terminal device decider 122 may also determine one or more terminal devices 200 scheduled to transmit the test request reception message only to one terminal device 200 corresponding to the test requesting person or the testing staff member designated by the server device 3.

According to another exemplary embodiment, the terminal device decider 122 may also determine one or more terminal devices 200 scheduled to transmit the test request reception message on the basis of various kinds of information related to the testing staff member and information related to the terminal devices (201 to 203) of the testing staff member. For this purpose, the terminal device decider 122 may read the storage 130 embedded in the medical device 100.

In addition, the terminal device decider 122 may assign priority to each of the terminal devices (201 to 203) when transmitting the test request reception message to each of the terminal devices (201 to 203). The test request reception messages may be sequentially transmitted to the plurality of terminal devices (201 to 203) according to the assigned priorities. A detailed description thereof will hereinafter be given.

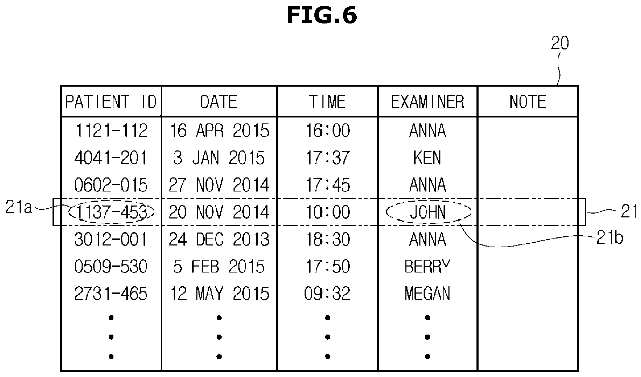

FIG. 6 is a conceptual diagram illustrating a method for deciding a terminal device according to whether or not medical testing is pre-performed, according to an exemplary embodiment.

Referring to FIG. 6, according to an exemplary embodiment, the terminal device decider 122 may select the terminal device 200 in a manner that the testing staff member who has performed the medical test on the patient to be tested, can re-test the patient at a later time. In this case, the terminal device decider 122 may determine a terminal device (e.g., a first terminal device 201) used by the testing staff member who has tested a specimen of the same patient to be tested among the plurality of terminal devices (201 to 203), to be a terminal device scheduled to transmit the test request reception message.

In more detail, as shown in FIG. 6, the terminal device decider 122 may call test history information 20 constructed using the legacy test information, may compare an examiner identification (ID) number (e.g., 1137-453) of the test request message with a patient ID number stored in the test history information 20, and may detect test information 21 having a same ID number 21a from the test history information 20. Subsequently, the terminal device decider 122 may obtain information 21b regarding the testing staff member (i.e., examiner) contained in the detected test information 21, and may obtain information regarding the terminal device corresponding to the obtained examiner, such that the terminal device decider 122 may select the terminal device 201 used by the examiner who has tested the specimen of the same patient from the plurality of terminal devices (201 to 203).

Upon receiving information regarding the plurality of examiners, the terminal device decider 122 may select the plurality of terminal devices (e.g., the first terminal device 201 and the second terminal device 202), and may also select any one terminal device 201 used by the examiner who has tested the specimen of the same patient, from the plurality of terminal devices (201 to 203) on the basis of the presence or absence of the latest test or the number of legacy tests.

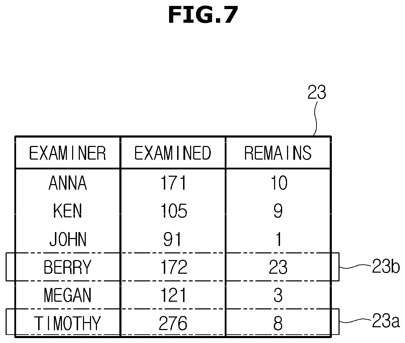

FIG. 7 is a conceptual diagram illustrating a method for deciding a terminal device according to a number of medical tests or a number of residual tests, according to an exemplary embodiment.

According to another exemplary embodiment, the terminal device decider 122 may determine at least one terminal device 200 scheduled to transmit the request reception message on the basis of the number of legacy tests or the number of residual tests allocated to the examiner.

In more detail, the terminal device decider 122 may determine the terminal device 200 by reading various statistical data related to the pre-finished tests. For example, as shown in FIG. 7, the terminal device decider 122 may read a current state information list 23 comprised of the number of tests and/or information regarding residual tests, may select an examiner 23a who has performed the largest number of tests (examined tests), and may determine the terminal device 200 corresponding to the selected examiner 23a. Alternatively, the terminal device decider 122 may select another examiner 23b having the largest number of residual tests to be examined, and may then determine the terminal device 200 corresponding to the selected examiner 23b.

In addition, the terminal device decider 122 may determine one or more terminal devices among the plurality of terminal devices (201 to 203) according to work experience, age and/or gender of the examiner, day-off of the examiner, selection of a test requesting person, or predefined setting.

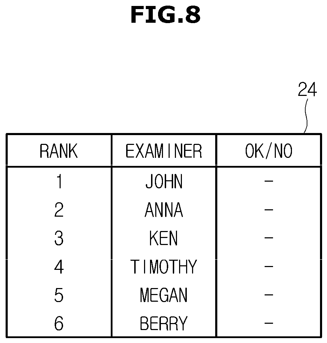

FIG. 8 is a conceptual diagram illustrating a list of medical testing staff member priorities according to a decision result, according to an exemplary embodiment.

The terminal device decider 122 may determine not only one terminal device 201 but also the plurality of terminal devices (201 to 203) to be the terminal device(s) scheduled to transmit the test request reception message, may assign priority information to respective determined terminal devices (201 to 203), and may generate a priority list 24 by constructing construct a list shown in FIG. 8.

In more detail, the terminal device decider 122 may use any one piece of information piece or combine plural pieces of information, such that it allocates unique priority information to respective terminal devices (201 to 203) according to the used or combined result.

In more detail, the terminal device decider 122 may automatically determine the above-mentioned priority information according to various kinds of information, for example, information indicating whether a target person (i.e., patient) to be tested has already been tested, the number of legacy tests applied to a patient to be tested, the number of residual tests allocated to the examiner, the number of legacy tests having been performed by the examiner, and work experience, age, or gender of the examiner. In this case, the terminal device decider 122 may also determine priority information by combining or using the above-mentioned information. For example, the terminal device decider 122 may allocate priority information to respective examiners in ascending numerical order of the number of residual tests, and may determine priority information of the respective terminal devices (201 to 203) corresponding to respective examiners according to priority information of the respective examiners.

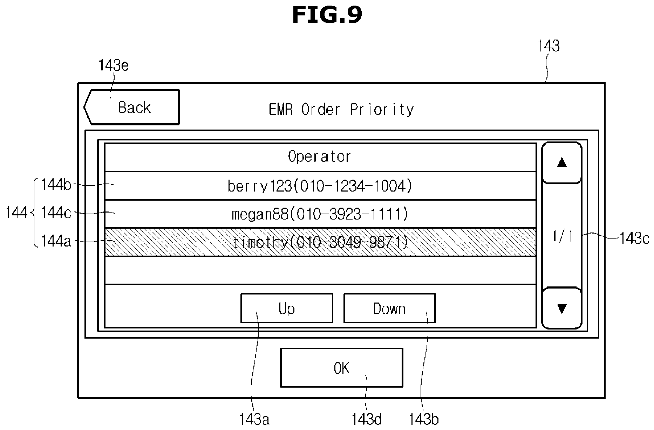

According to an exemplary embodiment, the terminal device decider 122 may also determine priority information of the respective terminal devices (201 to 203) according to the setting information predefined by a user. For example, the terminal device decider 122 may assign priority information to the respective examiners according to a user-defined order, for example, according to the order of a first examiner → a second examiner → a third examiner, and may determine priority information of the respective terminal devices (201 to 203) corresponding to the respective examiners according to priority information of the respective examiners. In more detail, the terminal device decider 122 may determine priority information on the basis of a user-established order of BERRY123 → MEGAN88 → TIMOTHY, as shown in FIG. 9.

The user, for example, the examiner or operator of the medical device, may manually establish or change the above-mentioned priority information.

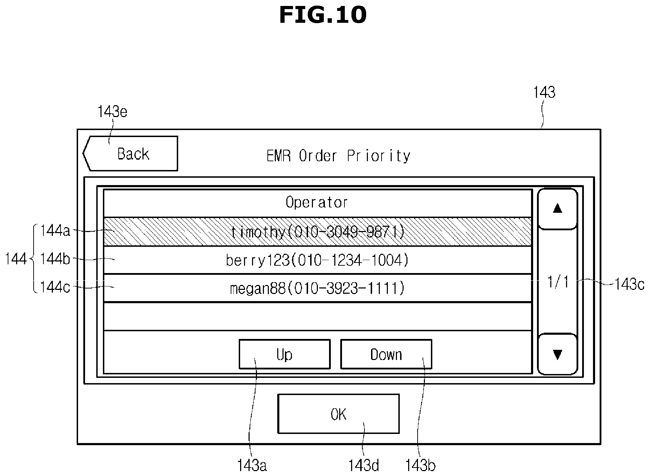

FIG. 9 is a conceptual diagram illustrating a first screen image through which medical testing staff member priority is manually changed, according to an exemplary embodiment. FIG. 10 is a conceptual diagram illustrating a second screen image through which medical testing staff member priority is manually changed, according to an exemplary embodiment.

Referring to FIGS. 9 and 10, assuming that the user desires to change priority information, the user may input a command for calling a setting screen image 143 used to change priority information, and the display 142 may display the setting screen image 143 used to change the priority information.

The setting screen image 143 for changing priority information may display a list in which the examiners (or operators) are sequentially arranged according to the predefined priority, or may display some parts 144 of the list. In addition, the setting screen image 143 may further display rank change buttons (143a, 143b) for changing ranks of examiners (or operators), a list change button 143c for displaying the remaining parts other than some parts of the displayed list, an accept button 143d for accepting change of the ranks of examiners, a cancel button 143e for cancelling the change of the ranks of examiners, etc. The above-mentioned setting screen image 143 may be designed in various ways according to selection of a system designer.

As shown in FIG. 9, a first examiner (or operator) 144b, a second examiner 144c, and a third examiner 144a may be sequentially displayed in the direction of an upper end to a lower end of the screen image 143. In this case, the position of each examiner (144a to 144c) may denote priority information of each examiner (144a to 144c). The user may select any one examiner (e.g., the third examiner 144a) from the list 144 through a mouse click or a touch action. In this case, to correctly inform the user of the selected examiner 144a, a part for displaying the selected examiner 144a thereon may be displayed in a distinctive color different from those of the other parts, or a circle or a check mark may further be displayed on the part for displaying the selected examiner 144a or in the vicinity of the part.

Thereafter, the user may manipulate the rank change buttons (143a, 143b) through a mouse click or a touch action, or may drag an image (e.g., an icon or an image located at a region in which the third examiner 144a is displayed) indicating the third examiner 144a, such that the position of the third examiner 144a may be shifted. Accordingly, the position of the third examiner 144a is changed to another position so that the third examiner 144a is displayed at the changed position, and the positions of unselected examiners (e.g., the first examiner 144b and the second examiner 144c) are also changed so that the first examiner 144b and the second examiner 144c are displayed at the changed positions according to the changed position of the selected examiner 144a. For example, as shown in FIG. 10, the selected examiner 144a may move to the highest position of the list 144 according to manipulation of the rank change button 143a, and unselected examiners (144b, 144c) may sequentially move to a position below the selected examiner 144a. Thereafter, assuming that the user manipulates the accept button (denoted by 'OK') 143d, the arrangement order of the third examiner 144a, the first examiner 144b, and the second examiner 144c is fixed, such that the priorities are fixed in the order of the third examiner 144a → the first examiner 144b → the second examiner 144c.

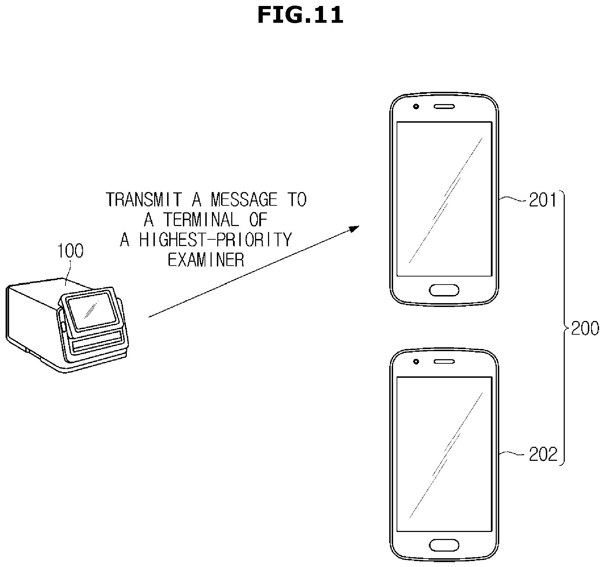

FIG. 11 is a conceptual diagram illustrating a method for transmitting a test request reception message to a mobile terminal device, according to an exemplary embodiment.

Referring to FIG. 11, the message generator 123 may generate a test request reception message corresponding to the test request message transmitted through the communication interface 110. The message generator 123 may decide the terminal device 200 scheduled to receive the message, and may generate the test request reception message at the same time or at different times. For example, the message generator 123 may generate a test request reception message before the terminal device decider 122 determines the terminal device 200 scheduled to receive the message, or may generate a test request reception message after the terminal device decider 122 determines the terminal device 200 scheduled to receive the message.

The test request reception message may include at least one among patient information, specimen information, information regarding the requested test, information regarding the prestored test, information regarding the test order, information regarding a medical device status, information indicating the presence or absence of an urgent test, information regarding the test requesting person, information regarding the examiner (or operator), and information regarding an estimated standby time. In this case, information regarding the test order may include information regarding the order of newly requested tests and/or the order of prestored tests. Information regarding medical device state information may include information as to whether the medical device can be tested, information as to whether the medical device is being used by another examiner, information as to whether the medical device is in an operation ready state such as preheating, information as to whether a defective or faulty part occurs in the medical device, and/or information as to whether various additional functions are being carried out. The medical device status information may be received from the status decider 125, and the estimated standby time information may be received from the estimated standby time calculator 124.

The test request reception message generated by the message generator 123 may be transmitted to the terminal device 200 through the communication interface 110. In this case, the test request reception message may be transmitted to any one terminal device, for example, the first terminal device 201, or may be transmitted to the plurality of terminal devices (201 to 203).

According to an exemplary embodiment, the medical device 100 may sequentially transmit the test request reception message to the plurality of terminal devices (201 to 203) according to priority information decided by the terminal device decider 122. In more detail, as shown in FIG. 11, the medical device 100 may initially transmit the test request reception message to one terminal device (e.g., the first terminal device 201) of the highest priority examiner among the plurality of terminal devices 200. In this case, the test request reception message may not be transmitted to another terminal device (e.g., the second terminal device 201) of a lower-priority examiner as compared to the first terminal device 201.

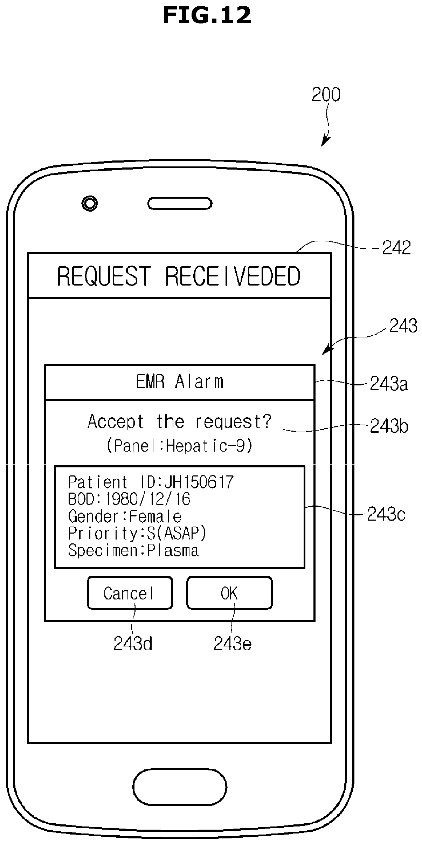

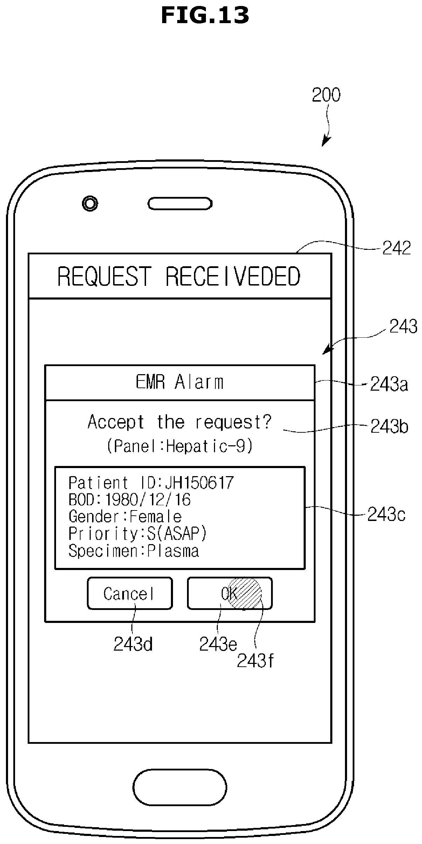

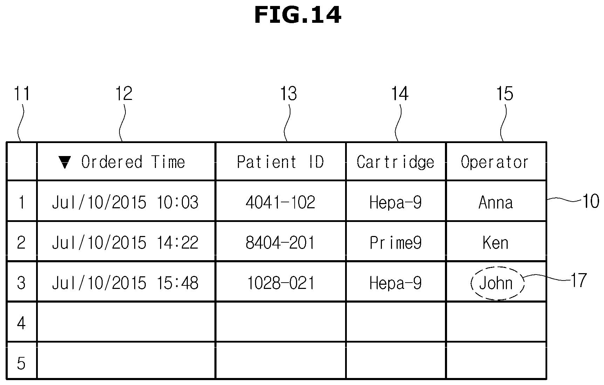

FIG. 12 is a conceptual diagram illustrating a method for displaying a test request reception message to a mobile terminal device, according to an exemplary embodiment. FIG. 13 is a conceptual diagram illustrating a method for allowing a medical testing staff member (examiner) to accept a test request, according to an exemplary embodiment. FIG. 14 is a conceptual diagram illustrating a list of medical test requests when a medical testing staff member (examiner) accepts a test request, according to an exemplary embodiment.

If the test request reception message is transmitted to the terminal device 200, the display 242 of the terminal device 200 may display a screen image 243 corresponding to the received test request reception message. In this case, the display 242 may display, for example, the exemplary screen image 243 corresponding to the test request reception message formed in a popup window shape.

The screen image 243 corresponding to the test request reception message may include a category or title 243a of the displayed message, an inquiry sentence or image 243b indicating reception or non-reception of the request, various kinds of information 243c related to the test request, a rejection button 243d (denoted by 'Cancel'), and an accept button 243e (denoted by 'OK'). In this case, various kinds of information 243c related to the test request displayed on the screen image 243 may include all kinds of information contained in the received test request reception message, and may include only specific information, which has been selected according to setting information predefined by either the user or the system designer, from the above various kinds of information contained in the test request reception message. According to selection of the user or the system designer, some parts of the above information may herein be omitted. In addition, other elements other than the above-mentioned elements may further be displayed on the screen image 243 according to selection of the user or the system designer.

If the user attempts to perform testing after investigating contents displayed on the screen image 243, i.e., if the user accepts the test request, the user may perform selection 243f by handling the accept button (OK) 243e. The user may perform selection 243f of the accept button 243e using a mouse click, a touch action, or other input interfaces according to the category of the terminal device 200.

If the accept button 243e is selected as shown in 243f, the terminal device 200 may transmit the accept message to the medical device 100, and the test list generator 121 of the medical device 100 may update the test request list 10 in response to the received accept message. In more detail, as shown in FIG. 14, the test list generator 121 may additionally input an ID code of the terminal device 200 having transmitted the accept message to the empty space 16 of the test request list 10, or may additionally input an ID code 17 of the operator (examiner) corresponding to the terminal device 200 having transmitted the accept message to the empty space 16 of the test request list 10, such that the operator (examiner) can be registered in the test request list 10. Through the above-mentioned processes, the test list generator 121 may update the test request list 10, and may store the updated test request list 10 in the storage 130.

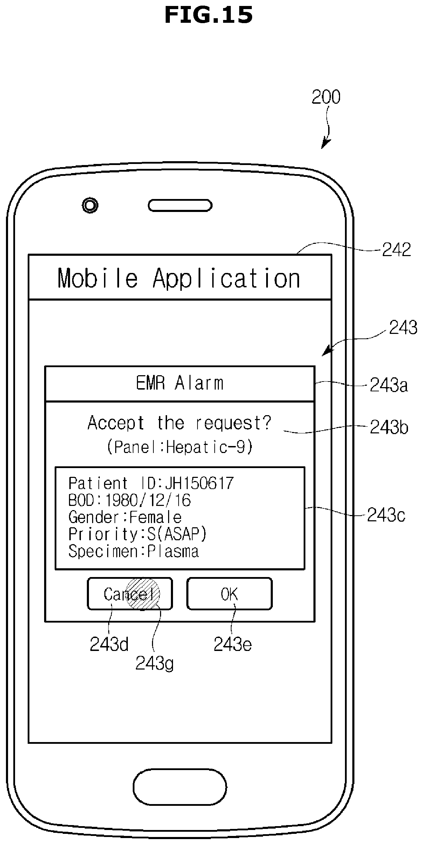

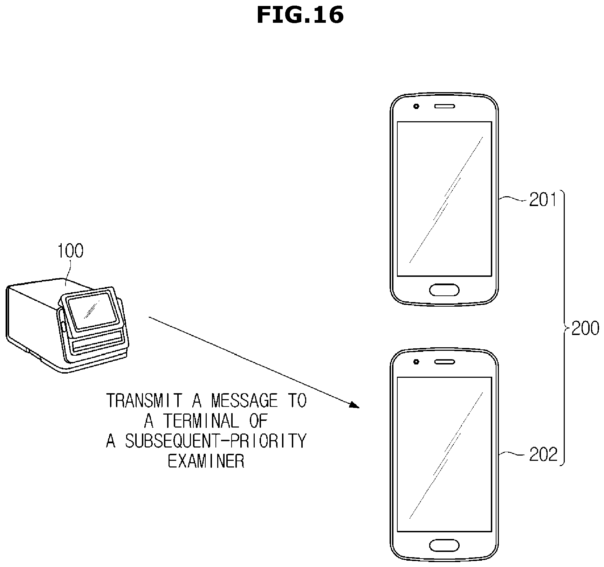

FIG. 15 is a conceptual diagram illustrating a method for allowing a medical testing staff member (examiner) to reject a test request, according to an exemplary embodiment. FIG. 16 is a conceptual diagram illustrating a method for transmitting a test request reception message to a second mobile terminal device according to rejection of a test request, according to an exemplary embodiment.

If the screen image 243 corresponding to the test request reception message is displayed, the user may confirm the displayed content and may not perform testing according to the confirmed result. In other words, the user may cancel the test request. In this case, as shown in FIG. 15, the user may manipulate the rejection button 243d using a mouse click, a touch action or other input interfaces, or may select the rejection button 243d as shown in 243g using a mouse click, a touch action or other input interfaces.

If the rejection button 243d is manipulated as shown in 243g, the terminal device 200 may transmit a rejection message to the medical device 100. The medical device 100 may receive the rejection message, and may transmit the test request reception message to a new terminal device (e.g., the second terminal device 202), as shown in FIG. 16. According to an exemplary embodiment, the rejection message may further include at least one additional message related to a cancel (or rejection) reason, unique item, or the like. The additional message related to the cancel reason or unique item may be directly written by the user of the terminal device 200.

According to an exemplary embodiment, the terminal device decider 122 of the controller 120 may additionally determine a new terminal device (e.g., the second terminal device 202) scheduled to transmit the test request reception message. If the second terminal device 202 is additionally determined as described above, the terminal device decider 122 of the controller 120 may perform such decision after excluding the first terminal device 201 having received the test request reception message. The medical device 100 may transmit the test request reception message to the newly decided terminal device 202.

According to another exemplary embodiment, the terminal device decider 122 may determine another terminal device scheduled to transmit the test request reception message according to the above-mentioned priority information. In more detail, as shown in the priority list 22 of FIG. 8, assuming that the terminal device decider 122 receives the rejection message from the first terminal device after transmitting the test request reception message to the first terminal device 201 (i.e., JOHN's terminal device of FIG. 8), the terminal device decider 122 may transmit the test request reception message to the second terminal device 202 (i.e., ANNA's terminal device of FIG. 8) corresponding to the next rank.

If the medical device 100 transmits the test request reception message to another terminal device 202, the additional message received from one terminal device (e.g., the first terminal device 201) having transmitted the rejection message may also be transmitted to the other terminal device 202. The user who carries the second terminal device 202 corresponding to the second rank may further confirm the additionally transmitted message, and may thus determine acceptance or denial of the test request according to the confirmed result.

Upon receiving the accept message from the second terminal device 200 having secondly received the test request reception message, the test list generator 121 of the controller 120 may additionally input either an ID code of the second terminal device 202 or an ID code of the examiner corresponding to the second terminal device 202 to the empty space 16 of the test request list 10, thereby updating the test request list 10 and storing the updated test request list 10 in the storage 130.

Upon receiving the rejection message from the second terminal device 202, the test list generator 121 may transmit the test request reception message to the third terminal device 203 (i.e., KEN's terminal device) corresponding to the third rank. As a result, the medical device 100 may receive the accept message or the rejection message from the third medical device 203, may update the test request list 10 as described above, or may transmit the test request reception message to the fourth terminal device (i.e., TIMOTHY's terminal device) corresponding to the fourth rank.

If the medical device 100 receives the rejection message from all the terminal devices (201 to 203) capable of transmitting the test request reception message, the examiner may be determined according to the predefined setting. For example, the medical device 100 may finally determine either a default examiner or the highest priority examiner to be an examiner for the requested test. The medical device 100 additionally inputs either the ID code of the confirmed examiner or the ID code of the terminal device of the confirmed examiner to the empty space 16 of the above-mentioned test request list 10, thereby updating the test request list 10 and storing the updated test request list 10 in the storage 130. In addition, the medical device 100 may transmit the test request reception message to the terminal device of the decided examiner. In this case, the above-mentioned operation for allowing the examiner to select any one among the accept message and the reject message regarding the test request may be disallowed.

According to an exemplary embodiment, if the terminal device decider 122 of the controller 120 does not receive at least one among the accept message and the reject message of the test request from the first terminal device 201 from the first terminal device 201 during a predefined time, another terminal device (e.g., the second terminal device 202) scheduled to transmit the test request reception message may be additionally determined as described above. The medical device may transmit the test request reception message to the newly decided second terminal device 202.

According to an exemplary embodiment, after the medical device 100 receives the accept message of the test request from the first terminal device 201, the medical device 100 may also transmit the test request reception message to another terminal device (e.g., the terminal device 202).

In more detail, after the controller 120 of the medical device 100 receives the accept message of the test request from the first terminal device 201, assuming that testing is not carried out during a predetermined time, the test request reception message may be transmitted to the terminal device (e.g., the second terminal device 202) of the next-rank examiner decided by the terminal device decider 122. In this case, the controller 120 may first determine whether the test corresponding to the test request is possible using the status decider 125. Although the medical device 100 is in a test available state, if the test is not started after lapse of a predefined time, the controller 120 may transmit the test request reception message to the second terminal device 202. Therefore, even when the examiner of the first terminal device 201 does not perform the test, the medical device 100 can allow another examiner to quickly perform testing.

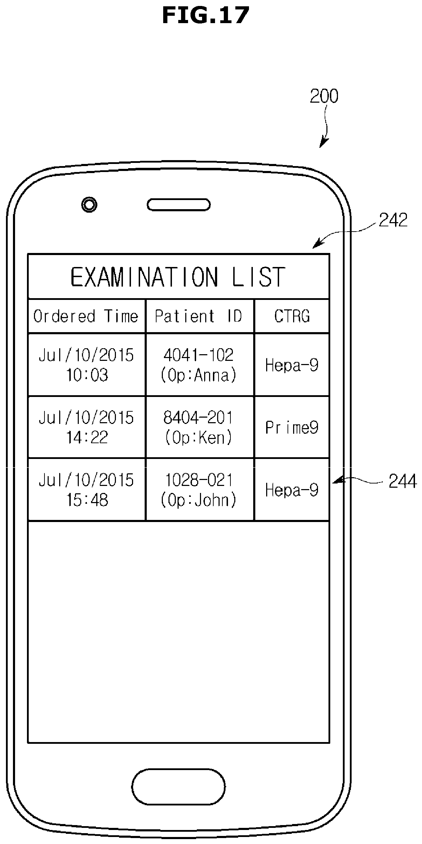

FIG. 17 is a conceptual diagram illustrating a method for checking a list of medical tests using a mobile terminal device, according to an exemplary embodiment.

Referring to FIG. 17, assuming that the test request list 10 is generated and updated by the test list generator 121 and is then stored in the storage 130 as described above, the user (e.g., the examiner) may read the test request list 10 stored in the storage 130 through the terminal device 200 as shown in FIG. 17. In this case, the terminal device 200 may transmit a list read request message to the medical device 100, and the medical device 100 may transmit the test request list 10 stored in the storage 130 to the terminal device 200 through the communication interface 110. The terminal device 200 may display a test request list 244 received from the medical device 100 for user recognition through the display 242.

According to an exemplary embodiment, the medical device 100 may also transmit all or some test request information of the test request list 10 to the terminal device 200. For example, the medical device 100 may transmit only test request information of the examiner corresponding to the terminal device 200 having received the list request message, among all the test request information stored in the test request list 10, to the terminal device 200, or may transmit only test request information of all examiners to the terminal device 200. In addition, the medical device 100 may transmit test request information generated during a predetermined time to the terminal device 200, or may transmit test request information regarding a patient, a test requesting person, or a specimen to the terminal device 200. The terminal device 200 may display the test request list 244 composed of only the received test request information for user recognition.

As described above, the test request list 10 may include an item regarding the test order 11. The test order 11 may indicate the execution order of plural tests. The test order 11 may be determined in various ways. For example, the test order 11 may be determined according to the reception order of the test request messages. The above-mentioned test order may be determined in various ways.

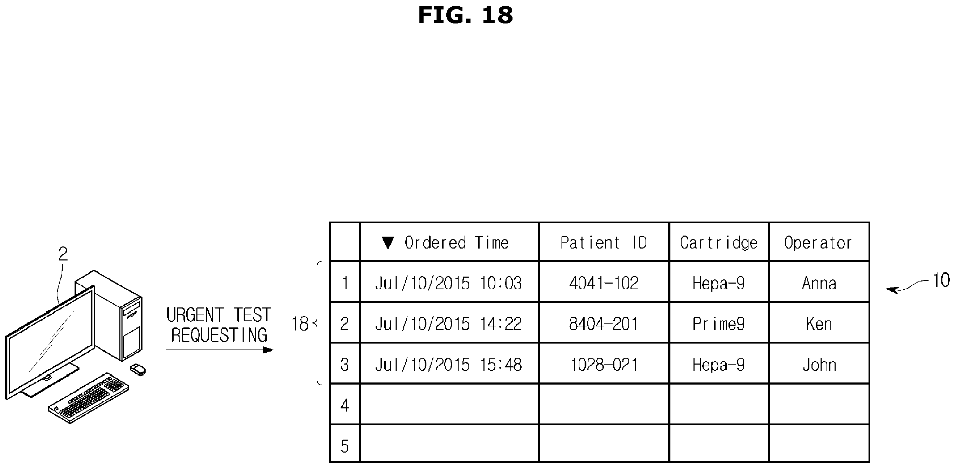

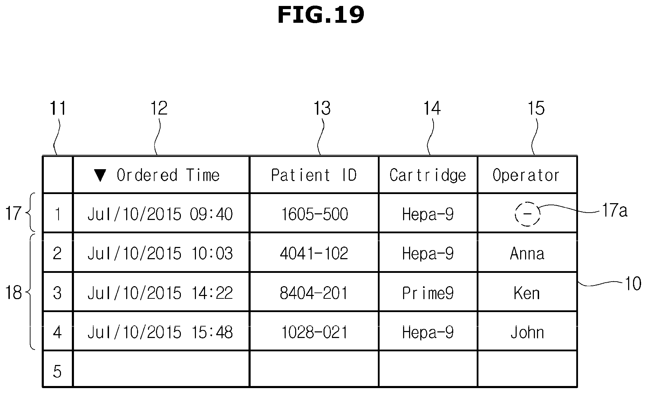

FIG. 18 is a conceptual diagram illustrating a first method for deciding an order of tests upon receiving an urgent test request, according to an exemplary embodiment. FIG. 19 is a conceptual diagram illustrating a second method for deciding an order of tests, according to an exemplary embodiment.

For example, assuming that the test request message to which the urgent test request is added is received from the computing device 2, test request information 17 corresponding to the test request message to which the urgent test request is added may be added to the test request list 10 in a manner that the urgent test can be performed earlier than prestored test request information 18, as shown in FIGS. 18 and 19. In other words, assuming that the test request list 10 includes legacy test request information pieces 18, the test list generator 121 may determine the test order 11 of the test request information corresponding to the newly received test request message to be the first test order 11, and may add the first test order 11 to the test request list 10, thereby generating or updating the test request list 10. In this case, because the examiner who is scheduled to perform the test corresponding to the test request message having the urgent test request is not yet determined, the examiner ID code 15 of the newly added test request information 17 may be an empty state 17a. The empty space 17a may be determined by the terminal device decider 122 in the same manner as described above.

As can be seen from FIG. 5, after the test request list 10 is generated, the examiner or operator who uses the medical device 100 may change the order of the requested test. In more detail, the examiner or operator who uses the medical device 100 may change the order of test request information stored in the test request list 10 such that the test order may be changed.

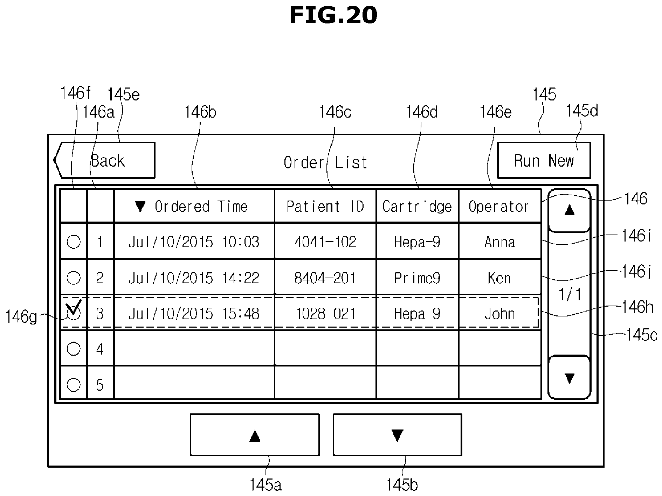

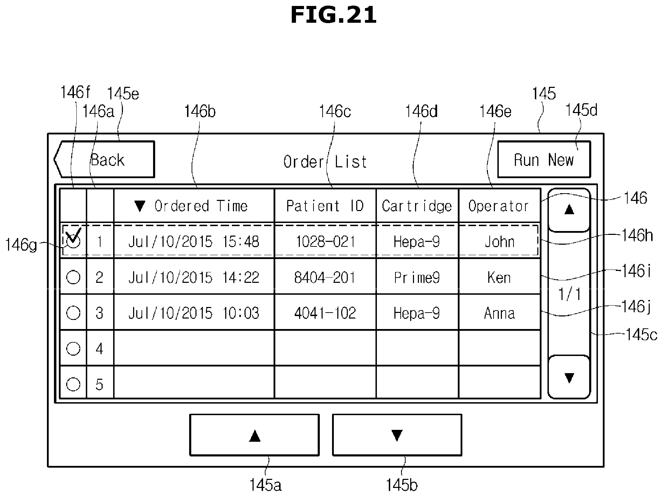

FIG. 20 is a conceptual diagram illustrating a method for changing an order of tests according to an exemplary embodiment. FIG. 21 is a conceptual diagram illustrating a method for changing an order of tests according to an exemplary embodiment.

According to an exemplary embodiment, the examiner or operator who uses the medical device 100 may manipulate the input interface 141 of the medical device 100. If the display 142 is a touchscreen, the examiner or operator may change the test order by manipulating the touchscreen.

Referring to FIG. 20, the display 142 of the medical device 100 may display a setting screen image 145 for changing the test order, such that the user can view the displayed screen image 145.

The setting screen image 145 may display a test request list 146 thereon, and the test request list 146 may include a plurality of test request information parts (146h to 146j). The plural test request information parts (146h to 146j) may be sequentially arranged from an upstream direction to a downstream direction of the setting screen image 145 according to the order of tests. In this case, the test request information 146i located in the upstream direction may be test request information regarding the test to be performed earlier than the test request information 146j located below the test request information 146i.

The test request information parts (146h to 146j) may include various items, for example, a test order 146a, a test request time 146b, a patient ID number 146c, a disc-shaped or cartridge-shaped reaction portion 146d, an ID code 146e of the examiner who is scheduled to perform testing, etc. In the same manner as described above, respective items contained in respective test request information parts (146h to 146j) may be selected by the user or the system designer, or may be added, deleted, or changed. The setting screen image 145 may further include order change buttons (145a, 145b) for changing the test order, a list change button 145c for further displaying other parts other than some parts of the display list, an accept button 145d for accepting the test order change, and a cancel button 145e for cancelling the test order change, such that the above-mentioned respective buttons can be displayed on the setting screen image 145. In addition, the setting screen image 145 may further include a selection or non-selection display space 146f capable of displaying the test request information parts (146h to 146j) selected as denoted by a V-marked dotted box 146g. The above-mentioned setting screen image 145 may be designed in various ways according to selection of the system designer.

The user may select any one (e.g., the third test request information 146h) of the plurality of test request information parts (146h to 146j). In this case, the user may select the third test request information 146h by mouse-clicking or touching a display region of the third test request information 146h or the selection or non-selection display space 146f. If the third test request information 146h is selected, the V-shaped mark is displayed on the selection or non-selection display space 146f, such that the user is notified of selection of the third test request information 146h. Of course, the medical device 100 may inform the user of a selection or non-selection state of the third test request information 146h by changing a current color of the display region of the third test request information 146h to another color or using various other methods.

Thereafter, the user may move the position of the third test request information 146h by touching or clicking the order change buttons (145a, 145b) or by dragging an image located at the display region of the third test request information 146h. Accordingly, the position of the third test request information 146h is changed, and the positions of some test request information pieces (for example, the position of the first test request information 146i located at the same position as the changed position of the third test request information 146h, and the position of the second test request information 146j located below the changed position of the third test request information 146h) are also changed according to the changed position of the third test request information 146h. For example, as shown in FIG. 21, the selected third test request information 146h may move to the highest end of the test request list 146 according to manipulation of the order change buttons (145a, 145b), and unselected test request information (146i, 146j) may be sequentially shifted to a lower end of the selected third test request information 146h. The order of pre-requested tests may be changed according to the above-mentioned method, such that the medical device 100 may perform a test corresponding to a test request message received at a relatively late time, prior to execution of the other test corresponding to the pre-received test request message.

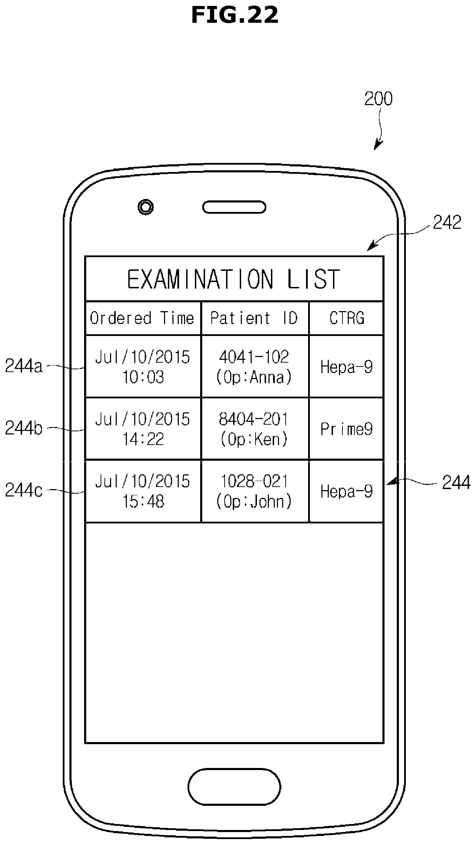

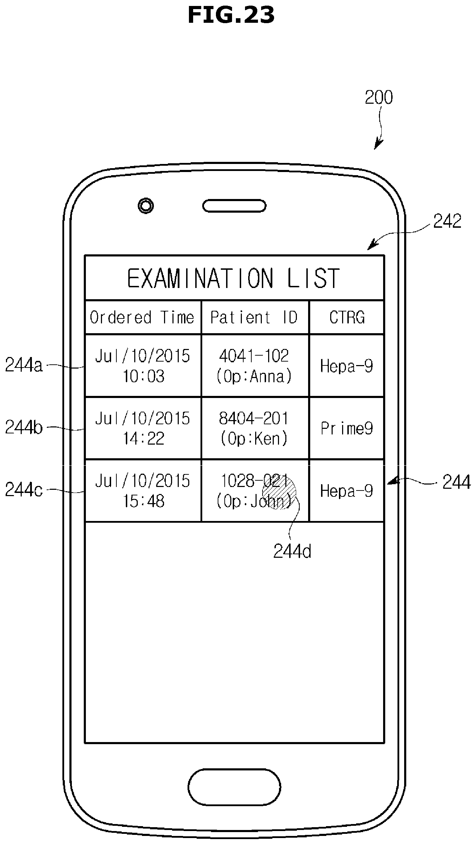

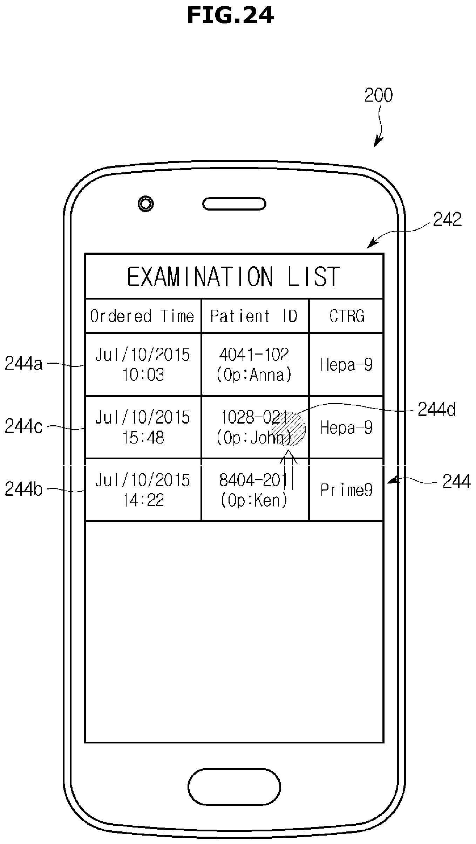

FIG. 22 is a conceptual diagram illustrating a first method for changing an order of tests using a mobile terminal device, according to an exemplary embodiment. FIG. 23 is a conceptual diagram illustrating a second method for changing an order of tests using a mobile terminal device, according to an exemplary embodiment. FIG. 24 is a conceptual diagram illustrating a third method for changing an order of tests using a mobile terminal device, according to an exemplary embodiment.

According to another exemplary embodiment, the examiner or operator who uses the medical device 100 may manipulate the input interface 241 of the terminal device 200 or may touch the display 242 implemented as a touchscreen, thereby changing the order of tests.

Referring to FIG. 22, the test request list 244 composed of a plurality of test request information parts (244a to 244c) may be displayed on the display 242 of the terminal device 200. As a result, the user may select any one (for example, the third test request information 244c) of the test request information parts (244a to 244c) displayed on the screen image through touching 244d, mouse clicking, or manipulation of the input interface such as a track pad or trackball. If the third test request information 244c is selected, the user may manipulate a separate input interface or may drag an image located at a display region of the third test request information 244c in a predetermined direction (i.e., in an upstream direction), thereby moving the position of the third test request information 244c. Therefore, the position of the third test request information 244c is changed, and the position of predetermined test request information (e.g., the second test request information 244b) may also be changed according to position change of the third test request information 244c. If the position of test request information (244b, 244c) is changed, the terminal device 200 may transmit a message regarding position change of the test request information (244b, 244c) to the medical device 100, and the terminal device 100 may change the test order in response to the position change of the test request information (244b, 244c). According to an exemplary embodiment, after the terminal device 200 generates the test order change message in response to the position change of the test request information (244b, 244c), the terminal device 200 may transmit the generated test order change message to the medical device 100, and the medical device 100 may change the test order according to the test order change message. The order of requested tests may also be changed according to the above-mentioned method, such that the medical device 100 may perform a test corresponding to a test request message received at a relatively late time, prior to execution of the other test corresponding to a test request message received at a relatively earlier time.

Referring to FIG. 3, the controller 120 may further include an estimated standby time calculator 124, and the estimated standby time calculator 124 may calculate an estimated standby time for each estimated test stored in the medical device 100, such that the estimated standby time for a test can be calculated. The calculated estimated standby time may be transmitted to the terminal device 200 and then displayed by the terminal device 200. Alternatively, the calculated estimated standby time may be displayed on the display 142 of the medical device 100 for user recognition. In addition, the estimated standby time calculator 124 may calculate the estimated standby time according to the predefined setting, or may calculate the estimated standby time according to a user's estimated standby time calculation command received from the terminal device 200 or entered by the input interface 141 of the medical device 100.

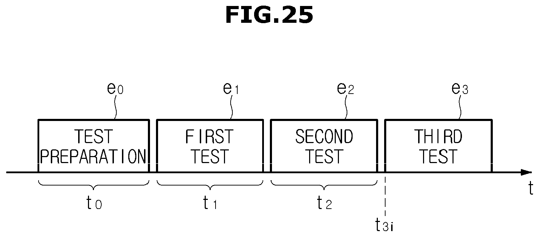

FIG. 25 is a conceptual diagram illustrating a first method for calculating an estimated standby time, according to an exemplary embodiment.

Referring to FIG. 25, assuming that the first to third tests (e1 to e3) are scheduled, and the estimated standby time (t3i) regarding the third test (e3) is calculated, the estimated standby time calculator 124 may estimate and calculate a time (t1) to be consumed for the scheduled first test (e1), and may estimate and calculate the other time (t2) to be consumed for the scheduled second test (t2). In this case, the estimated standby time calculator 124 may calculate the respective times (t1, t2) consumed for respective tests (e1, e2) according to categories of the respective tests (e1, e2). Thereafter, the estimated standby time calculator 124 may calculate the estimated standby time (t3i) of the third test (e3) on the basis of the sum (t1+t2) of the estimated consumption times (t1, t2). Therefore, as the number of estimated tests increases, or as the consumption time of each estimated test is elongated, the estimated standby time increases.

In this case, the estimated standby time calculator 124 further adds a time (t0) (for example, a preheating time or the like) used for test preparation (e0) of the medical device 100 to the sum (t1+t2) of the estimated consumption times (t1, t2), thereby calculating the estimated standby time (t3i) for the third test (e3). In addition, a test intermediate standby time consumed between the first test (e1) and the second test (e2) is estimated, and the estimated test intermediate standby time is further added to the estimated result, such that the estimated standby time (t3i) for the third test (e3) may be calculated. The calculated estimated standby time (t3i) may be transmitted to the terminal device 200 through the communication interface 200.

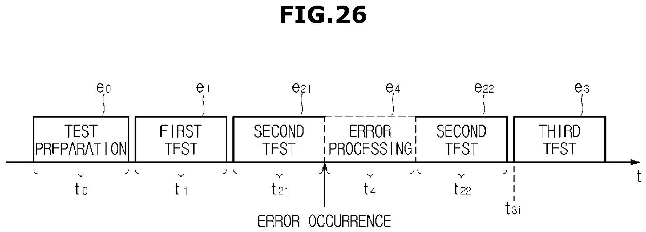

FIG. 26 is a conceptual diagram illustrating a second method for calculating an estimated standby time, according to an exemplary embodiment.

Referring to FIG. 26, an unexpected error may occur during the second test (e21). In this case, the estimated standby time calculator 124 may estimate a time (t4) used for error processing (e4). After the error has been resolved, a time (t22) consumed for the second test (e22) to be resumed is estimated and calculated, and a time (t4) used for error processing (e4) and the other time (t22) consumed for the second test (e22) are summed as denoted by (t4+t22), such that the estimated standby time (t3i) for the third test (e3) can be calculated. In this case, according to categories of generated errors, the estimated time (t22) consumed for the second test (e22) to be resumed may be equal to or shorter than the time (t2) consumed for the second test (e2) having no errors. The estimated standby time calculator 124 may confirm the generated error. If the estimated standby time calculator 124 has to completely perform the above test according to the confirmation result, the time (t2) consumed for the second test (e2) is set to the estimated time (t22) consumed for the second test (e22) to be resumed. If the test (e22) can resume after incompletion of the test (e21), the time (t21) consumed for the second test (e21) to be performed previously, is subtracted from the time (t2) consumed for the second test (e2) as denoted by (t2-t21), such that the estimated time (t22) consumed for the second test (e22) to be resumed can be calculated. The estimated standby time (t3i) for the third test (e3) may be transmitted to the terminal device 200. Assuming that the estimated standby time (t3i) is transmitted to the terminal device 200 prior to error occurrence, the medical device 100 may transmit not only information regarding the error occurrence but also a new estimated standby time (t3i) newly calculated according to such error occurrence to the terminal device 200.

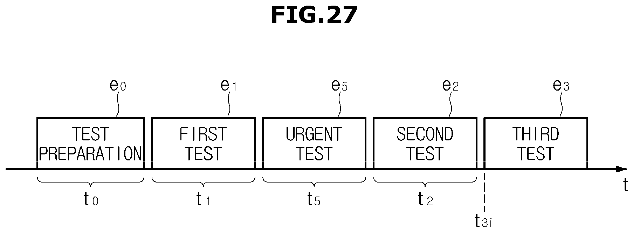

FIG. 27 is a conceptual diagram illustrating a third method for calculating an estimated standby time, according to an exemplary embodiment.

Referring to FIG. 27, assuming that the first to third tests (e1 to e3) are estimated and the estimated standby time (t3i) for the third test (e3) is calculated, if the urgent test (e5) is newly added, the estimated standby time calculator 124 may estimate a time (t1) consumed for the estimated first test (e1), a time (t2) consumed for the estimated second test (t2) and may estimate a time (t2) consumed for the newly added urgent test (e5), and the estimated consumption times (t1, t2, t5) are summed as denoted by (t1+t2+t5), such that the estimated standby time (t3i) for the third test (e3) can be calculated. As described above, the estimated standby time calculator 124 may further add the time (t0) used for test preparation (e0) of the medical device 100 to the sum (t1+t2+t5), may estimate the respective times consumed for the respective tests (e1, e2, e5), and may further add the estimated times to the estimated result, thereby calculating the estimated standby time (t3i). The estimated standby time (t3i) for the above-mentioned estimated third test (e3) may be transmitted to the terminal device 200. If the estimated standby time (t3i) is transmitted to the terminal device 200 prior to addition of the urgent test (e5), the medical device 100 may transmit not only the estimated standby time (t3i) newly calculated according to addition of the urgent test (e5) but also an addition message of the urgent test (e5) to the terminal device 200.

The status decider 125 of the controller 120 may determine a current state of the medical device 100. In more detail, the status decider 125 may determine whether the medical device 100 is in a test available state, is performing the test, or is in a test unavailable state. In this case, the test unavailable state of the medical device 100 may indicate information as to whether a faulty operation occurs in the medical device 100 such that the medical device 100 is unable to perform testing, information as to whether a sufficiently high power-supply voltage is not being supplied to the medical device 100, information as to whether the medical device 100 is in a preheating state or in a cleaning mode, or other state information indicating that other tests cannot be carried out. The status decider 125 may determine a current state using one or more sensors embedded in each component of the medical device 100. For example, the status decider 125 may detect the electrical signal generated from each component, and may determine a current state of the medical device 100 on the basis of the detected result.

The decision result of the status decider 125 may be transmitted to at least one terminal device 200 through the communication interface 110, and the user may confirm a current state of the medical device 100 through the screen image displayed on the display 242 of the terminal device 200.

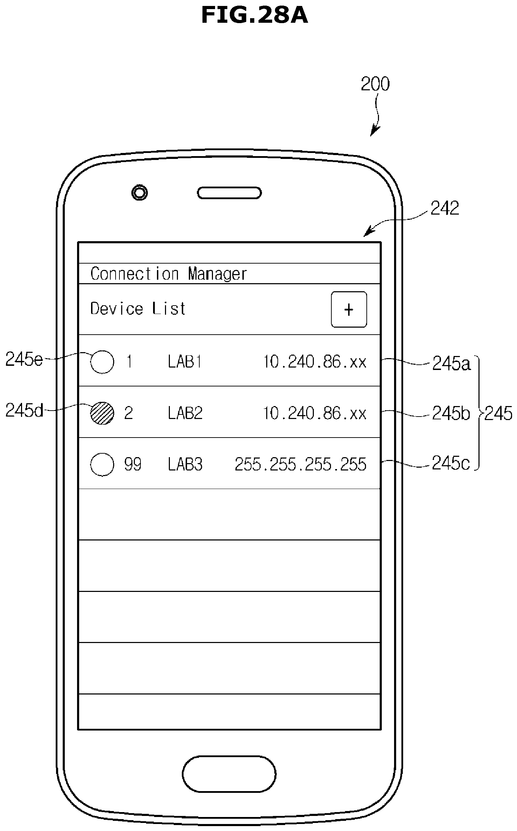

FIG. 28A is a conceptual diagram illustrating a method for checking states of a plurality of medical devices using a mobile terminal device, according to an exemplary embodiment. Referring to FIG. 28A, the display 242 of the terminal device 200 may display a state display screen image 245 for displaying a current state of at least one medical device 100 (for example, the first medical device 101 and/or the second medical device 102).

At least one medical device (101, 102) may be configured in the form of a list and then displayed on the state display screen image 245. In this case, the status displays (245d, 245e) for indicating states of the medical device may be mounted to information parts (245a to 245c) of the respective medical devices (101, 102) constructing the list of medical devices. The status displays (245d, 245e) may display states of the medical devices (101, 102) using letters, symbols and/or numbers. Alternatively, the status displays (245d, 245e) may display the status information of the medical devices (101, 102) using figures (e.g., a circle or square) or other images. The status displays (245d, 245e) may display the states of the medical device (101, 102) using predetermined colors in a manner that the user can intuitively recognize the status information of the medical devices. For example, the status display 245d may display a white figure when the medical device (e.g., the first medical device 101) is in an available state, and may display a red figure when the medical device (e.g., the second medical device 102) is in an unavailable state. The status display 245d may allow the user to intuitively recognize the states of the medical devices (101, 102) by viewing the color of each figure displayed on the status display 245d.

The authenticator 126 of the controller 120 may authenticate the terminal device 200 or may authenticate the user of the terminal device 200. In this case, the authenticator 126 may authenticate the terminal device 200 or the user of the terminal device 200 on the basis of the terminal device 200's information and the examiner information stored in the storage 130.

An exemplary method for correcting the list of test requests stored in each medical device using a mobile terminal device according to exemplary embodiments will hereinafter be described with reference to the attached drawings.

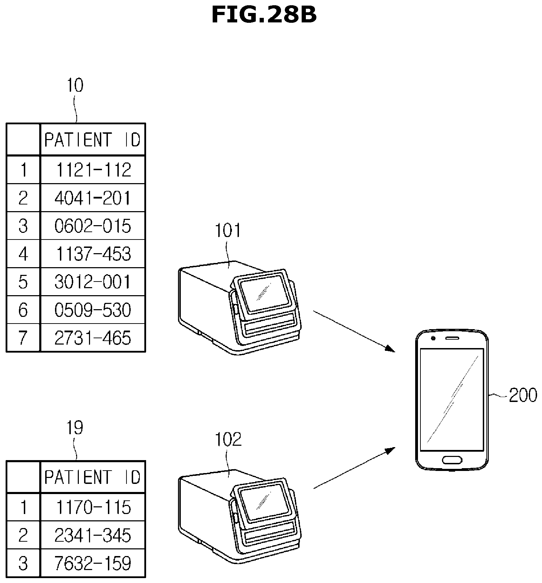

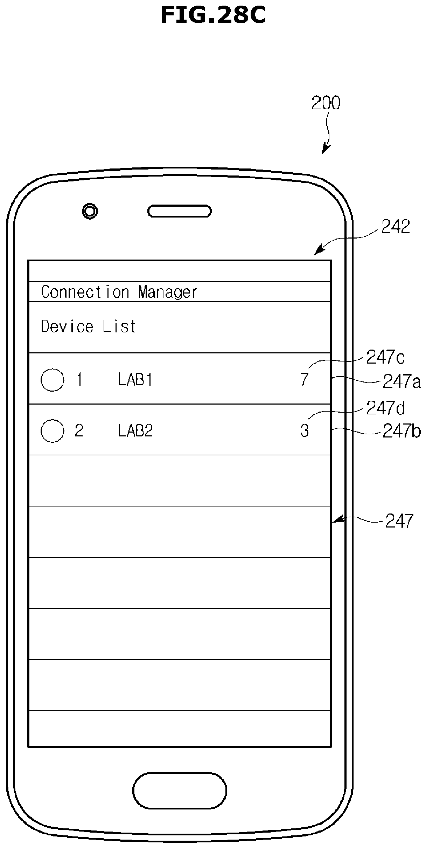

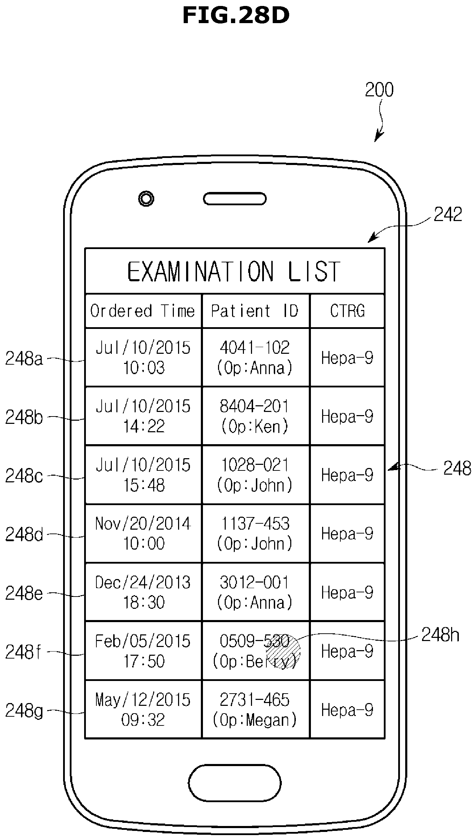

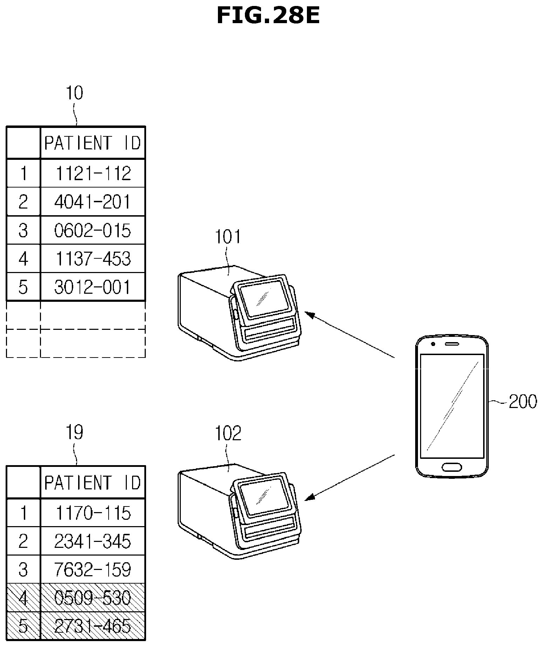

FIG. 28B is a conceptual diagram illustrating a method for correcting medical test request lists respectively stored in respective medical devices, according to an exemplary embodiment. FIG. 28C is a diagram illustrating a screen image for displaying a number of medical test request lists respectively stored in respective medical devices displayed on a mobile terminal device, according to an exemplary embodiment. FIG. 28D is a diagram illustrating a screen image for displaying a number of medical test request lists respectively stored in respective medical devices displayed on a mobile terminal device, according to an exemplary embodiment. FIG. 28E is a diagram illustrating a screen image for correcting medical test request lists, according to an exemplary embodiment.

Referring to FIG. 28B, the respective medical devices (101, 102) may generate test request lists (10, 19) obtained on the basis of the test request message received from at least one computing device 2 of the test requesting person, and may generate the test request lists (10, 19). In this case, the numbers of test request information pieces of the test request lists (10, 19) of the respective medical devices (101, 102) are equal to or different from each other. In addition, the number of test request information pieces of the test request list 10 of any one medical device 101 may be relatively higher or less than the number of test requests of the test request list 19. For example, the test request list of the first medical device 101 may include 7 test request information pieces, and the test request list of the second medical device 102 may include 3 test request information pieces. In this case, unbalance of the numbers of received test requests may cause inefficient use of the medical devices (10, 19). If the test requests are distributed as described above, the user may correct respective test requests of the medical devices (101, 102) using the terminal device 200 carried by the user, such that the respective test requests can be redistributed.

In more detail, as shown in FIG. 28B, the test request list 10 stored in the first medical device 100 and the other test request list 19 stored in the second medical device 102 may be confirmed. For example, the test request list 10 stored in the first medical device 100 or information regarding the test request list 10 may be transmitted to the terminal device 200 of the user, and the test request list 19 stored in the second medical device 100 and information regarding the test request list 19 may be transmitted to the terminal device 200 of the user.

The user may confirm the test request lists (10, 19) through the terminal device 200. In more detail, the display 242 of the terminal device 200 may display an screen image 247 for providing test request information stored in at least one medical device 100 (for example, the first medical device 101 and/or the second medical device 102) according to the received test request lists (10, 19).

In this case, as shown in FIG. 28C, the display 242 of the terminal device 200 may display information parts (247a, 247b) of the respective medical devices (101, 102) in the form of a list. Information parts (247a, 247b) regarding the respective medical devices (101, 102) may display not only symbols or letters capable of identifying medical devices (101, 102), but also the numbers (247c, 247d) of test requests currently estimated in the respective medical devices (101, 102). The user may manipulate the input interface 241 of the terminal device 200, or may touch display points of the information parts (247a, 247b) of the respective medical devices (101, 102), such that at least one among the medical devices (101, 102) can be selected.

As can be seen from FIG. 28D, if at least one among the medical devices (101, 102) is selected by the user, the terminal device 200 may display the test request list 10 stored in a medical device (for example, the first medical device 101) on an screen image 248. Accordingly, the test requests (248a to 248g) applied to the first medical device 101 may be displayed on the screen image 248. The user may manipulate the input interface 241 or may touch a display point 248h of the test request to be transmitted to the second medical device 102, such that the user may select at least one test request to be transmitted to the second medical device 102 among the plurality of test requests. For example, if at least one test request (for example, the 6th test request 248h and the 7th test request 248g) is selected, information corresponding to the 6th test request 248f and information corresponding to the 7th test request 248g may be transmitted to the second medical device 102.

Referring to FIG. 28E, the second medical device 102 may add the 6th test request 248f and the 7th test request 248g to the test list 19 using information regarding the 6th test request 248f and information regarding the 7th test request 248g. Therefore, the test request list 19 of the second medical device 102 is updated. In addition, the terminal device 200 may transmit specific information, indicating that the 6th test request 248f and the 7th test request 248g have been transmitted to the second medical device 102, to the first medical device 101. The first medical device 101 may delete the 6th test request 248h and the 7th test request 248g from the test request list 10 according to the received information. Therefore, the test request list 10 of the first medical device 101 can also be updated.

Through the above-mentioned process, the number of test requests stored in the first medical device 101 is reduced from 7 to 5, and the number of test requests stored in the second medical device 102 increases from 3 to 5, such that the test requests of the respective medical devices can be properly redistributed.

If the test request lists (10, 19) are updated, the respective medical devices (101, 102) may transmit the updated result to the terminal device 200. Transmission of the update result may be performed whenever such updating is completed, and may be performed at intervals of a predetermined time. In this case, the terminal device 200 configured to receive the update result from the respective medical devices (101, 102) may further include not only the terminal device used for redistribution of the test request but also terminal devices of other examiners. Therefore, even when an examiner transmits the test request from a medical device 101 to another medical device 102, the other examiner may easily confirm whether test requests have been redistributed through his or her terminal device (for example, the second terminal device 202). In addition, the update result may also be transmitted to the computing device 2, such that the test requesting person may also confirm whether the test requests have been redistributed.

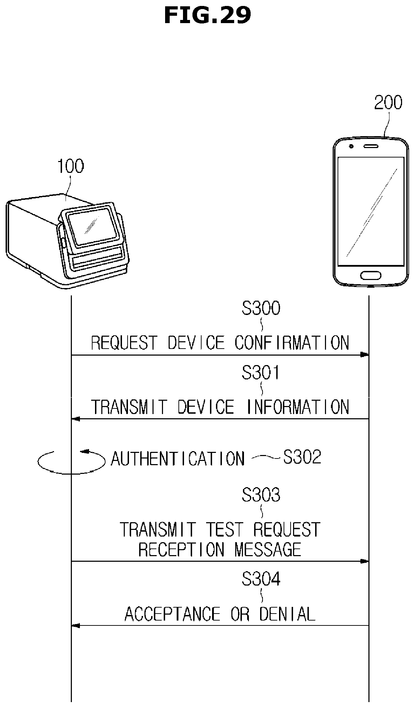

A method for authenticating the mobile terminal device according to exemplary embodiments will hereinafter be described with reference to FIG. 29.

FIG. 29 is a flowchart illustrating a method for authenticating a mobile terminal device, according to an exemplary embodiment.

According to an exemplary embodiment, the authenticator 126 may determine whether a terminal device configured to communicate with the medical device 100 is the terminal device 200 having authority to receive the test request reception message or has authority to transmit commands or information to the medical device 100.

In more detail, as shown in FIG. 29, assuming that the medical device 100 is connected to a predetermined terminal device 200 to communicate with the predetermined terminal device 200, the medical device 100 may first transmit a message regarding a device confirmation request through a wired communication network or a wireless communication network (S300). The operation for transmitting the device confirmation request message by the medical device 100 may be performed after reception of the test request message, or may also be performed prior to reception of the test request message. In addition, the operation for transmitting the device confirmation request message by the medical device 100 may be performed after the decision process of the terminal device 200 is performed by the terminal device decider 122, or may also be performed irrespective of the decision process of the terminal device 200.

The terminal device 200 may automatically transmit device information to the medical device 100, or may manually transmit the device information of the medical device 200 to the medical device 100 according to user manipulation (S301). The terminal device 200's information applied to the medical device 100 may include, for example, at least one among a phone number of the terminal device 200, an International Mobile Equipment Identity (IMEI), an Integrated Circuit Card Identifier (ICCID), an International Mobile Subscriber Identity (IMSI), an IP address, and a Bluetooth address.

The medical device 100 may authenticate the terminal device 200 on the basis of the received device information (S302). For example, the medical device 100 may read the terminal device information (e.g., the terminal device database) stored in the storage 130 of the medical device 100, and may search for specific data that is identical to or corresponds to the received device information, thereby authenticating the terminal device 200.

After successful authentication of the terminal device 200, the medical device 10 may temporarily or non-temporarily store information regarding the authenticated terminal device 200 therein.

The medical device 100 may transmit the test request reception message to the successfully authenticated terminal device 200 (S303). In this case, the terminal device decider 122 may determine at least one terminal device (e.g., the first terminal device 201) scheduled to transmit the test request reception message among at least one pre-authenticated terminal device 200. The medical device 100 may transmit the test request reception message to the first terminal device 201 according to decision of the terminal device decider 122. After transmission of the test request reception message, the user of the terminal device 200 may accept or reject the test request as described above as described above, and the terminal device 200 may transmit the acceptance or denial message to the medical device 100 according to user decision (S304).

According to another exemplary embodiment, the authenticator 126 may also authenticate whether the user who uses the terminal device 200 is a user (e.g., examiner) corresponding to the terminal device 200.

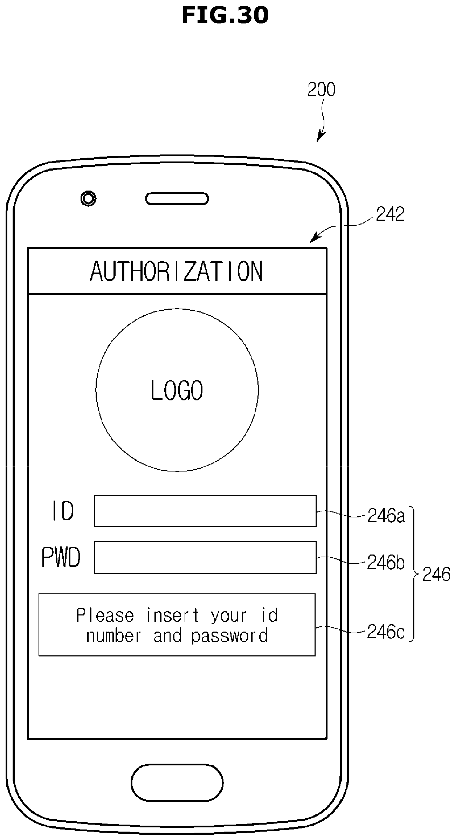

FIG. 30 is a diagram illustrating an authentication screen image displayed on a mobile terminal device, according to an exemplary embodiment.

Referring to FIG. 30, assuming that the medical device 100 is connected to the terminal device 200 to communicate with the terminal device 200, the authentication request message can be transmitted from the medical device 100 to the terminal device 200, and the terminal device 200 may display an authentication screen image 246 on the display 242 in response to the authentication request message, as shown in FIG. 30.

The authentication screen image 246 may display an input space 246a for displaying a user ID and another input space 246b for displaying a user password on the authentication screen image 246. In addition, the authentication screen image 245 may further display a request message 246c for requesting the user to input his or her ID and/or password. According to an exemplary embodiment, the ID input space 246a is omitted from the authentication screen image 246, and only the pass word input space 246b and the request message 246 may be displayed on the authentication screen image 246.

The ID and/or password entered by the user may be transmitted to the medical device 100, and the authenticator 126 may perform authentication by referring to the user list stored in the storage 130. For example, the authenticator 126 may determine the presence or absence of the same ID and/or password identical to the ID and/or password received from the user list stored in the storage 130, and may perform authentication according to the determined result.

After completion of such authentication, the medical device 100 may transmit the test request reception message or the like to the terminal device 200, or may calculate the estimated standby time according to a user command entered through the terminal device 200, or may change priority of the examiner. If authentication failure occurs, under the condition that the medical device 100 finishes communicating with the terminal device 200 or does not provide the terminal device 200 with information, the medical device 100 may be configured not to receive commands or information from the terminal device 200.

If user authentication is completed as described above, one user may receive the test request reception message from the medical device 100 not only through one terminal device but also through different terminal devices. Alternatively, the user may input a command for the medical device 100. In addition, if the user requests authentication using a new terminal device, information regarding a new terminal device may also be transmitted to the medical device 100. In this case, the medical device 100 may store information regarding the new terminal device therein, and the above-mentioned authentication process shown in FIG. 29 may also be performed on the new terminal device.

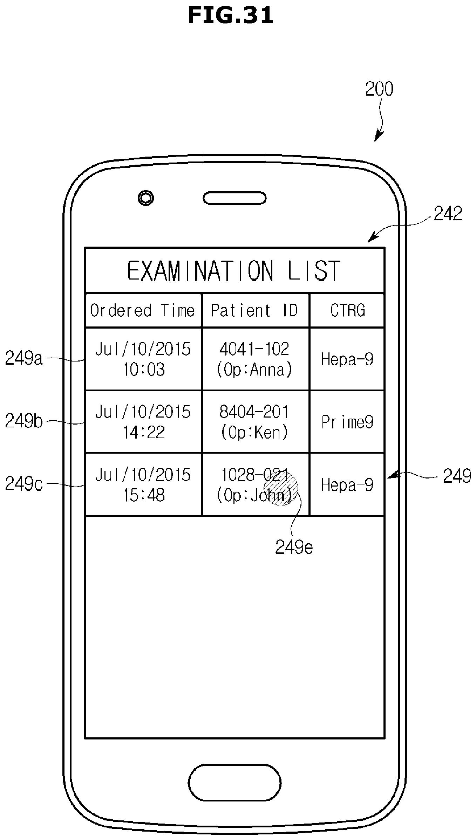

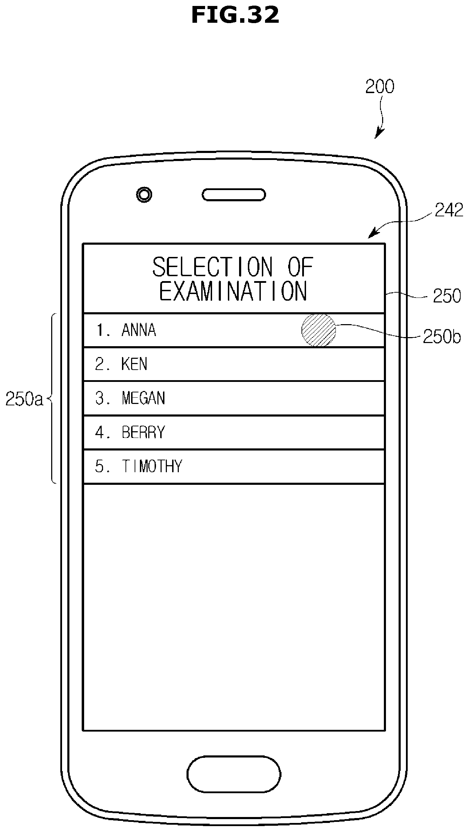

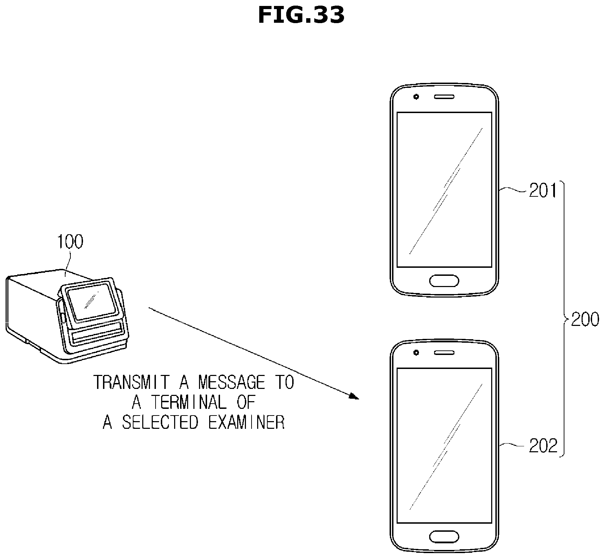

FIG. 31 is a conceptual diagram illustrating a first method for selecting a medical test to be requested for another medical testing staff member using a mobile terminal device, according to an exemplary embodiment. FIG. 32 is a conceptual diagram illustrating a second method for selecting a medical test to be requested for another medical testing staff member using a mobile terminal device, according to an exemplary embodiment. FIG. 33 is a conceptual diagram illustrating a third method for selecting a medical test to be requested for another medical testing staff member using a mobile terminal device, according to an exemplary embodiment.

According to an exemplary embodiment, the examiner may transmit a transmission command of the test request reception message to the medical device 100 using the terminal device 200 to transmit a requested test to another examiner, thereby controlling the medical device 100.

For example, as shown in FIG. 31, the first examiner manipulates the terminal device 200 in a manner that the display 242 of the terminal device 200 can display a screen image 249 for transmitting the test request. The test request list composed of at least one test request information part (249a to 249c) may be displayed on the screen image 249.

The first examiner may select test request information (e.g., the third test request information 249c) from the test request information parts (249a to 249c) by touching or clicking 249e the test request information or using an input interface such as a track pad. In this case, the first examiner may select test request information related to the first examiner, i.e., test request information accepted by the first examiner. Alternatively, the first examiner may select only test request information corresponding to the test request reception message applied to the first examiner.

The terminal device 200 may store the selected request information 249c in the storage 230, and the display 242 of the terminal device 200 may display an examiner selection screen image 250. The examiner display screen image 250 may include an examiner list 250a comprised of one or more examiners to which the test request reception message can be applied.

The first examiner may select any one examiner (for example, the second examiner denoted by ANNA) from the examiner display screen image 250 by touching or clicking the second examiner (ANNA) or through execution of other manipulations or the like, as can be seen from 250b. The terminal device 200 may store information regarding the selected examiner (ANNA) in the storage 230.

Subsequently, the terminal device 200 may transmit the selected test request information 249c and information regarding the selected examiner (ANNA) to the medical device 100. The medical device 100 may generate the test request reception message on the basis of the selected test request information 249c, and may determine a terminal device 202 scheduled to transmit the test request reception message on the basis of information regarding the selected examiner (ANNA). Thereafter, as shown in FIG. 33, the medical device 100 may transmit the test request reception message corresponding to the test request information 249c to the terminal device 202 of the selected examiner.

According to an exemplary embodiment, the examiner may also transmit a test request message to any one among the medical devices (101, 102) by manipulating the terminal device 200. In this case, the examiner may also transmit the test request message to the medical device (e.g., another medical device other than the first medical device 101) having transmitted the test request reception message.

In more detail, if the examiner may receive the test request reception message from the first medical device 101, and if it is determined that the test requested by the second medical device 102 be more preferable than the test requested by the first medical device 101, the examiner manipulates the terminal device 200 to generate a test request message corresponding to the test request reception message, and then transmits the test request message to the second medical device 102.

In this case, the examiner may first read the test request list stored in the first medical device 101, may select any one test request from the test request list, and may determine whether the test request message corresponding to the selected test request has been transmitted to the second medical device 102. If the second medical device 102 receives the test request message from the terminal device 200, the second medical device 102 performs processing of the received test request message in the same manner as in the test request message received from the computing device 2, such that the second medical device 102 may generate the test request list, may determine the terminal device decision, and may determine the test order.

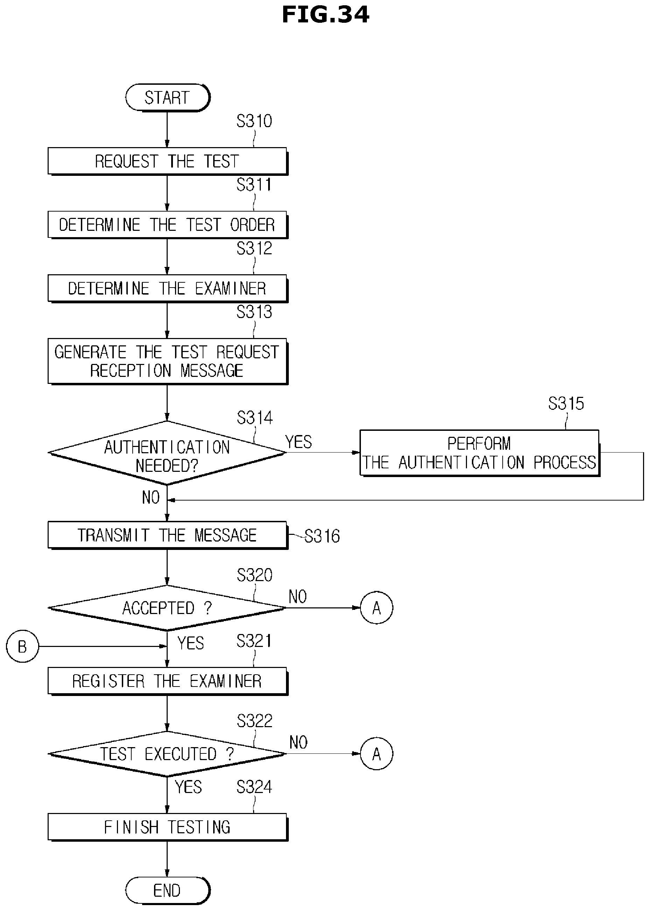

Medical test request control methods according to exemplary embodiments will hereinafter be described with reference to FIGS. 34 to 37.

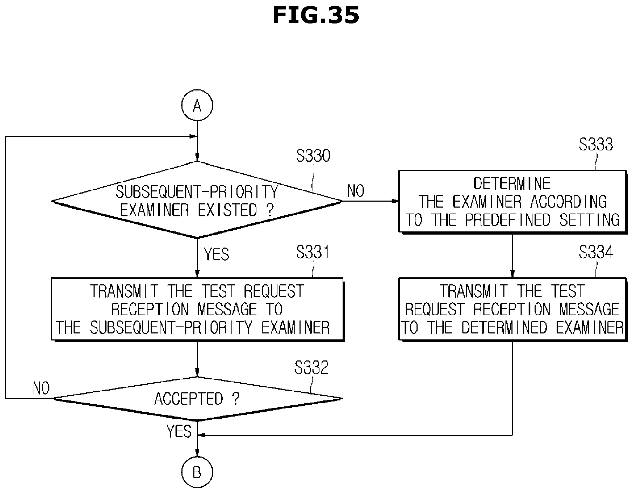

FIG. 34 is a flowchart illustrating a first method for controlling a medical test request, according to an exemplary embodiment. FIG. 35 is a flowchart illustrating a second method for controlling a medical test request, according to an exemplary embodiment.

Referring to FIG. 34, a test requesting person (e.g., a doctor or a nurse) may request testing (or examination) of a specimen of a target person (e.g., a patient) using the computing device (S310). The computing device generates a test request message according to manipulation of the test requesting person, and transmits the generated test request message to the medical device. In this case, the generated test request message may also be transmitted to the medical device through the server device. In this case, the medical device may be an IVD (In Vitro Diagnosis) medical device, or may be an image diagnosis medical device. Additionally, various medical devices used in medical sites (e.g., hospitals) may be used as the above-mentioned medical devices. In addition, the test request message may include various kinds of test related information. In this case, the various kinds of test related information may include at least one among information regarding the patient, information regarding the specimen, information regarding the requested test, information regarding the presence or absence of an urgent test, information regarding the test requesting person, and information regarding the examiner.

The medical device may receive the test request message, and may use a newly transmitted test request. Alternatively, the medical device may determine the test order using the legacy requested test and the newly transmitted test request, and may generate the test request list according to the determined order (S311).

In this case, the test order may be determined according to the transmission order of test orders, i.e., according to the reception order of the test request message. However, if a new test request is an urgent test request, the order of the new test request may be determined in a manner that the new test request can be performed earlier than another test request.

The test order decided by the medical device may be changed by the user, for example, the examiner or the administrator of the medical device. The user may change the test order by manipulating a user interface of the medical device, or may change the test order using the terminal device communicating with the medical device. In this case, the terminal device may be a mobile terminal device. If the test order is decided, the medical device may determine the examiner scheduled to perform the requested test and/or the terminal device of the examiner (S312).

According to an exemplary embodiment, the medical device may confirm content of the test request message. If the test request message includes information regarding the examiner who desires to perform testing, the examiner and the terminal device of the examiner can be determined on the basis of the resultant test request message. According to another exemplary embodiment, the medical device may read a history of tests (or examinations) of the examiner, and thus determine the examiner who has performed a legacy patient and a terminal device of the examiner. According to another exemplary embodiment, the medical device may confirm the number of legacy tests of the examiner or the number of residual tests, and may then determine the examiner and the terminal device of the examiner. According to an exemplary embodiment, the medical device may determine the history, age, or gender of the examiner, information regarding day-off of the examiner, or the predefined setting.

If there are plural examiners, the medical device may assign different priorities to the respective examiners. In other words, the medical device assigns unique priority to the respective communicable terminal devices, the test request reception message may be transmitted to the terminal device according to priority information. The medical device may assign unique priority to respective terminal devices using various methods. For example, the medical device may assign unique priority to each terminal device according to the predefined priority, may assign unique priority to each terminal device according to content of the test request message, or may assign unique priority to each terminal device using the test history of the examiner, the number of tests, or the number of residual tests, etc.

According to an exemplary embodiment, the examiner determining operation (S312) may be performed after the above-mentioned test order determining operation (S311), as shown in FIG. 34. Alternatively, according to another exemplary embodiment, the examiner determining operation (S312) may also be performed prior to the test order determining operation (S311). Additionally, the examiner determining operation S312 may also be performed simultaneously with the test order determining operation S311.

If the examiner is decided, the medical device may also generate the test request reception message (S313). The test request reception message may include at least one among information regarding the patient, information regarding the specimen, information regarding the requested test, information regarding the prestored test, information regarding the requested test, test order information regarding the prestored test, information regarding a medical device state, information regarding the presence or absence of an urgent test, information regarding the test requesting person, and information regarding the examiner.

The test request reception message generating operation S 131 may be performed after the test order determining operation S311 and the examiner determining operation S312, or may also be performed before the test order determining operation S311 or after the examiner determining operation S312. In addition, the test request reception message generating operation S313 may also be performed after the authentication operations (S314, S315).

If the medical device is connected to at least one terminal device to communicate with the at least one terminal device, and if authentication is to be performed (S314), the medical device may perform the authentication process (S315). As described above, the authentication process S315 may be a process for authenticating the terminal device, or may be another process for authenticating the user of the terminal device.

The authentication necessity determining operation S314 and the authentication operation S315 may be performed before the test request transmitting operation S310, or may also be performed before the message sending operation S316 as shown in FIG. 34. In addition, the authentication necessity determining operation S314 and the authentication operation S315 may be performed at various time points according to selection of the system designer.

After decision of the test order (S311), if the examiner and the terminal device scheduled to transmit the test request reception message are determined, or if priority information of the examiner and/or the terminal are determined (S312), and if the test request reception message is generated (S313), the medical device may transmit the test request reception message to the determined terminal device (S316). Assuming that priority of the examiner and/or priority the terminal device are determined, the medical device may transmit a test request reception message to the terminal device having the highest priority. In this case, the number of terminal devices decided by the medical device may be singular or plural. As described above, the medical device may transmit the test request reception message to the terminal device over a wired communication network or a wireless communication network.

The terminal device may receive the test request reception message, and may display the screen image corresponding to the received test request reception message on the display embedded in the terminal device. The display may be implemented as a touchscreen. In this case, the display may also function as the input interface. The screen image corresponding to the test request reception message may include a sentence or image inquiring about acceptance or denial.

The user (e.g., the examiner) of the terminal device may confirm the screen image corresponding to the test request reception message, and may determine acceptance or denial of the confirmed message by manipulating the input interface embedded in the terminal device or by touching the touchscreen (S320).

If the user of the terminal device accepts the received message (S320 'Yes'), acceptance information is transmitted to the medical device, and the medical device may register the accepted user as the examiner of the received test request (S321). In more detail, the medical device may input an ID code of the terminal device having transmitted the acceptance message or an ID code of the examiner corresponding to the terminal device having transmitted the acceptance message to the empty spaces of the examiner item contained in the test request list, thereby updating and storing the test request list.

If the user of the terminal device does not accept the received message (S320 'No'), as shown in FIG. 35, the medical device may determine the presence or absence of an examiner who has the second priority and/or the presence or absence of a terminal device of the second-priority examiner in the priority list (S330). If the second-priority examiner and/or the second-priority terminal device are present in the priority list, the medical device may transmit the test request reception message to the terminal device of the second-priority examiner (S331). The medical device may perform the above-mentioned operation even when the acceptance or denial message is not received from the user of the terminal device during a predetermined time. In this case, not only the denial message but also an additional message entered by the user of the terminal device may also be transmitted to the medical device. The medical device may also transmit the additional message along with the test request reception message to the terminal device of the second-priority examiner.

The second-priority examiner may determine acceptance or denial through his or her terminal device (S332). If the second-priority examiner accepts the test request, acceptance information is transmitted to the medical device. The medical device may register the second-priority examiner according to the acceptance information, thereby updating the test request list (S321).

If the user of the second-priority terminal device does not accept the test request (S332 'No'), the medical device may determine the presence or absence of an examiner who has the third priority and/or the presence or absence of a terminal device of the third-priority examiner (S330) in the priority list (S330). If the third-priority examiner and/or the third-priority terminal device are present in the priority list, the medical device may transmit the test request reception message to the terminal device of the third-priority examiner (S331). The above-mentioned process may be repeated before any one among the examiners contained in the list accepts the test request or until the subsequent-priority examiner or the subsequent-priority terminal device is not present (S330 'No').

If the subsequent-priority examiner and/or the subsequent-priority terminal device are not present (S330 'No'), the medical device may determine the examiner according to the predefined setting (S333). For example, the medical device 100 may determine a predetermined default examiner or the highest-priority examiner to be an examiner for the requested test, and may update the test request list on the basis of the determined result. In this case, the medical device may transmit the test request reception message to the terminal device of the determined examiner such that it can allow the examiner to recognize the fact that the examiner is determined to be the examiner of the requested test (S334). In this case, the operation for allowing the examiner to select any one among acceptance and denial of the test request may be prohibited.

After the examiner is input to and registered in the test request list (S321), the examiner may test the specimen by manipulating the medical device (S322 'Yes'), and may then finish testing after completion of the test result (S324). The obtained test result may be transmitted to the computing device and/or the server device of the test requesting person over a wired communication network or a wireless communication network, and the computing device and/or the server device may store the received test result therein. The test requesting person may confirm the test result using the computing device and/or the server device.

On the other hand, according to an exemplary embodiment, the medical device may determine whether the test is being carried out (S322). If the examiner does not perform testing within a predetermined time (S322 'No'), the medical device may transmit the test request reception message to the next priority terminal device (S330, S331). The next priority examiner may input an acceptance or denial message of the test request by manipulating the terminal device. The next-priority examiner may be registered in the test request list according to the acceptance or denial message of the next-priority examiner (S321), or the test request reception message is transmitted to the terminal device of the subsequent-priority examiner (S330, S331). As described above, if the subsequent-priority examiner and/or the subsequent-priority terminal device are not present (S330 'No'), the medical device may determine the examiner according to the predefined setting, and thus update the test request list. The medical device transmits a predetermined message to the terminal device of the determined examiner so that the determined examiner can recognize the fact that he or she has been determined to be an examiner of the requested test (S334).

The medical test request control method may further include transmitting the test request message corresponding to the test request reception message from one medical device to another medical device. In this case, the medical device having received the test request message corresponding to the test request reception message may perform the above-mentioned operations (S310 to S324), such that an examiner can perform the requested test.

Besides, the medical test request control method may include controlling the medical device to determine a current status thereof (e.g., information as to whether the medical device can be used or not), and transmitting status information of the medical device to the terminal device through a communication interface. In this case, the user may determine a current status (e.g., information as to whether the medical device can be used or not) using the terminal device. Transmitting the status information may be performed, for example, before the test execution operations (S322, S324).

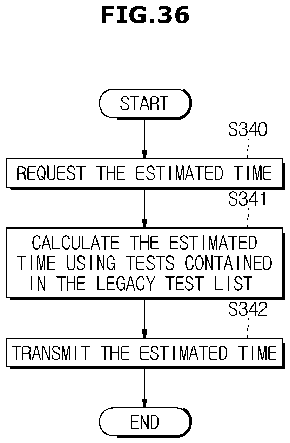

FIG. 36 is a flowchart illustrating a method for calculating an estimated standby time, according to an exemplary embodiment.

Referring to FIG. 36, the user, for example, the examiner or administrator of the medical device may manipulate the terminal device or may directly manipulate the medical device, such that the user can request provision of the estimated standby time (S340).

Upon receiving the estimated standby time provision request, the medical device may calculate the estimated standby time by using test information and the legacy test request list (S341). For example, the medical device may estimate a consumption time for each estimated test, may sum the respective estimated consumption times, and may thus calculate the estimated standby time. In this case, a time used for test preparation or a test intermediate standby time may be added to calculate the estimated standby time. In addition, the medical device may also calculate the estimated standby time on the basis of either information regarding transmission or non-transmission of an urgent test request or information regarding status information of the medical device (e.g., information as to whether the medical device can be used or not). A detailed description thereof has already been disclosed with reference to FIGS. 25 to 27, and as such a detailed description thereof will herein be omitted for convenience of description.

If the estimated standby time is calculated, the medical device may transmit the estimated standby time to the terminal device so that the terminal device may display the estimated standby time thereon or may also display the estimated standby time on the display of the medical device (S342).

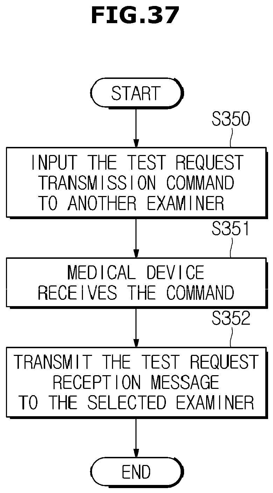

FIG. 37 is a flowchart illustrating a method for transmitting a test request reception message to another test staff member, according to an exemplary embodiment.

Referring to FIG. 37, the examiner may control the medical device using the terminal device in such a manner that information regarding the requested test can be transmitted to another examiner.

In more detail, the examiner may manipulate the terminal device, and thus input a command regarding transmission of the test request reception message to the terminal device of another examiner (S350). In this case, prior to the test request reception message transmission command, the examiner may select the other examiner and a requested test to be transmitted to the other examiner.

The medical device may receive the test request reception message transmission command applied to the terminal device of the other examiner (S351), and may transmit the test request reception message regarding the selected test to the selected user's terminal device contained in the received command (S352).

The other examiner may analyze content displayed on the display in response to the test request reception message, and may determine acceptance or denial (S320 of FIG. 34 or S332 of FIG. 35).

In addition, the exemplary embodiments may also be implemented through computer-readable code and/or instructions on a medium, e.g., a computer-readable medium, to control at least one processing element to implement any above-described embodiments. The medium may correspond to any medium or media that may serve as a storage and/or perform transmission of the computer-readable code.

The computer-readable code may be recorded and/or transferred on a medium in a variety of ways, and examples of the medium include recording media, such as magnetic storage media (e.g., ROM, floppy disks, hard disks, etc.) and optical recording media (e.g., compact disc read only memories (CD-ROMs) or digital versatile discs (DVDs)), and transmission media such as Internet transmission media. Thus, the medium may have a structure suitable for storing or carrying a signal or information, such as a device carrying a bitstream according to one or more exemplary embodiments. The medium may also be on a distributed network, so that the computer-readable code is stored and/or transferred on the medium and executed in a distributed fashion. Furthermore, the processing element may include a processor or a computer processor, and the processing element may be distributed and/or included in a single device.

As is apparent from the above description, the medical device, the system and method for controlling the medical test request, and the program stored in a recording medium according to exemplary embodiments can allow a medical testing staff member located at a remote site to quickly receive/confirm a medical testing request, such that medical testing can be properly and rapidly carried out.

The above-mentioned medical device, the system and method for controlling the medical test request, and the program stored in a recording medium according to exemplary embodiments can transmit a medical testing request to an appropriate testing staff member. If the testing staff member having received the test request is unable to perform medical testing, a test request can be transferred to a new testing staff member, such that the medical testing of the hospital can be efficiently distributed and performed.

The foregoing exemplary embodiments are examples and are not to be construed as limiting. The present teaching can be readily applied to other types of apparatuses. Also, the description of the exemplary embodiments is intended to be illustrative, and not to limit the scope of the claims, and many alternatives, modifications, and variations will be apparent to those skilled in the art.

## Claims

1. A medical apparatus comprising:
a communication unit configured to receive at least one test request message; and
a control unit configured to generate a test request reception message in response to the at least one test request message,
wherein the communication unit transmits the test request reception message to at least one mobile terminal device.

2. The medical apparatus according to claim 1, wherein the test request reception message includes at least one of information regarding a target object to be tested, information regarding a specimen, information regarding a requested test, information regarding a pre-stored test, information regarding a test order of the requested test and the pre-stored test, information regarding a status of the medical apparatus, information regarding an urgent test request, information regarding a test requesting person, and information regarding an examiner who conducts a test.

3. The medical apparatus according to claim 1, wherein the control unit determines a test order on the basis of the at least one test request message, and changes the test order according to information regarding the urgent test request or a test order change command received from the mobile terminal device.

4. The medical apparatus according to claim 1, wherein the control unit further calculates an estimated standby time based on a number of estimated tests and a category of an estimated test, or based on the number of the estimated tests, the category of the estimated test and status information of the medical apparatus, or corrects the test order according to an urgency degree of each test, and calculates the estimated standby time according to the corrected result.

5. The medical apparatus according to claim 1, wherein:
the control unit determines the mobile terminal device scheduled to transmit the test request reception message; and
the communication unit transmits the test request reception message to the determined mobile terminal device.

6. The medical apparatus according to claim 5, wherein:
the control unit determines the mobile terminal device scheduled to transmit the test request reception message according to a priority of each examiner; and
the communication unit transmits the test request reception message to a high-priority mobile terminal device.

7. The medical apparatus according to claim 5, wherein the control unit determines the priority according to a test history of each examiner, a number of tests conducted by each examiner, a number of residual tests of each examiner, or predetermined priority information.

8. The medical apparatus according to claim 7, wherein the communication unit receives a request acceptance message or a request denial message from the mobile terminal device.

9. The medical apparatus according to claim 8, wherein:
if the communication unit receives the request denial message from the mobile terminal device, or if a response signal from the mobile terminal device is not generated for a predefined time, the control unit controls the communication unit to transmit the test request reception message to a subsequent-priority mobile terminal device, or
if the communication unit receives the request acceptance message from the mobile terminal device, and if testing is not started for a predetermined time, the control unit controls the communication unit to transmit the test request reception message to a subsequent-priority mobile terminal device.

10. The medical apparatus according to claim 8, wherein the request denial message includes an additional message entered by a user of the mobile terminal device.

11. The medical apparatus according to claim 1, wherein:
the communication unit receives a transmission command of the test request reception message from the mobile terminal device to another mobile terminal device; and
the control unit controls the communication unit to transmit the test request reception message to the other mobile terminal device according to the received command.

12. The medical apparatus according to claim 1, wherein the mobile terminal device receives the test request reception message, and transmits the test request message corresponding to the test request reception message to another medical device.

13. The medical apparatus according to claim 1, wherein the communication unit receives the at least one test request message from a personal computer (PC), a server device, or the at least one mobile terminal device.

14. The medical apparatus according to claim 1, wherein the control unit determines specific information as to whether it is impossible to use the medical apparatus, and transmits the specific information to the at least one mobile terminal device through the communication unit.

15. A method for controlling a medical test request, comprising:
receiving, by a medical apparatus, at least one test request message;
generating, by the medical apparatus, a test request reception message in response to the at least one test request message;
transmitting, by the medical apparatus, the test request reception message to at least one mobile terminal device.
